# EUROPÄISCHE PATENTSCHRIFT

(11) **EP 3 292 563 B1**
(45) Veröffentlichungstag und Bekanntmachung des Hinweises auf die Patenterteilung: **08.11.2023**
(21) Anmeldenummer: 16721783.5
(22) Anmeldetag: 04.05.2016
(51) Int. Cl.: H01J 49/04, G01N 33/18

(54) **ON-LINE MASSENSPEKTROMETER ZUR ECHTZEITERFASSUNG FLÜCHTIGER KOMPONENTEN AUS DER GAS- UND FLÜSSIGPHASE ZUR PROZESSANALYSE**
ONLINE MASS SPECTROMETER FOR REAL-TIME DETECTION OF VOLATILE COMPONENTS FROM THE GAS AND LIQUID PHASE FOR PROCESS ANALYSIS
SPECTROMÈTRE DE MASSE EN LIGNE POUR LA DÉTECTION EN TEMPS RÉEL DE COMPOSANTS VOLATILES PROVENANT DE LA PHASE GAZEUSE ET LIQUIDE AUX FINS D'ANALYSE DE PROCESSUS

(30) Priorität: 05.05.2015 DE 102015208250
(43) Veröffentlichungstag der Anmeldung: 14.03.2018
(73) Patentinhaber: Fraunhofer-Gesellschaft zur Förderung der angewandten Forschung e.V., 80686 München (DE)
(72) Erfinder: JOOS, Martin, 76189 Karlsruhe (DE); STIER, Matthias, 73529 Schwäbisch Gmünd (DE); SCHERLE, Stephan, 71364 Winnenden (DE)
(74) Vertreter: Schrell, Andreas
(86) Internationale Anmeldenummer: PCT/EP2016/060055
(87) Internationale Veröffentlichungsnummer: WO 2016/177810

(56) Entgegenhaltungen:
- WO-A1-2009/146396
- WO-A2-2005/104177
- DE-B3- 10 319 130
- GB-A- 2 392 114
- Anonymus: "Foto der Einlass-Systeme des Spektrometers Finnigan MAT95", , 21. April 2011 (2011-04-21), XP055284920, Gefunden im Internet: URL:https://web.archive.org/web/2011042110 4620/http://www.chemgapedia.de/vsengine/vl u/vsc/de/ch/3/anc/masse/ms_einlass_vakuum. vlu/Page/vsc/de/ch/3/anc/masse/2_massenspe ktrometer/2_1_einlasssystem/anschaul/foto_ alle_m39ht0402.vscml.html [gefunden am 2016-06-30]

## Beschreibung

Die vorliegende Erfindung betrifft ein Verfahren zum massenspektrometrischen Analysieren von in einer flüssigen und einer gasförmigen Probe vorhandenen Substanzen sowie eine Vorrichtung zum Durchführen dieses Verfahrens.

Prozessmassenspektrometer zur Analyse einer Gasphase sind aus dem Stand der Technik bekannt, beispielsweise von Thermo Scientific und Extrel CMS, LLC.

Darüber hinaus offenbart DE 41 33 300 A1 ein "Online"-Massenspektrometer mit Membraneinlass, mit dem flüchtige Bestandteile einer wässrigen Lösung mit einer Empfindlichkeit im unteren ppb-Bereich (ppb = "parts per billion", also ein Milliardstel) erfasst werden können. Gemäß dem in DE 41 33 300 A1 offenbarten Verfahren kann die flüssige Phase über eine Kapillare kontinuierlich mittels einer pulsationsarmen Pumpe in einen Membraneinlass geleitet, an einer PTFE-Membran im Inneren vorbeigeführt und über eine weitere Kapillare entweder wieder zurück zum Ausgangspunkt geführt oder verworfen werden. Auf der Permeatseite des Membraneinlasses liegt ein Vorvakuum an, wodurch kontinuierlich die Flüssigkeit an der Membranoberfläche verdampft wird. Wird ein Drehventil geöffnet, gelangt ein Teil des im Vorvakuum, auch als Feinvakuum bezeichnet, befindlichen Gasstromes in ein Hochvakuum und wird dort als Ionenstrom im Massenspektrometer gemessen.

Die WO 2009/146396 A1 offenbart einen Massenspektrometer, der über einen Einlass zur Untersuchung von gasförmigen Proben aus der Gaschromatographie und über einen zusätzlichen Probeneinlass verfügt, wobei eine in den Probeneinlass eingebrachte Probenlösung zu einem Spray aus Flüssigkeitstropfen zerstäubt wird und das Spray anschließend in einem Evaporator oder mit einem Heizelement verdampf wird.

Aus dem Stand der Technik ist jedoch kein Massenspektrometer bekannt, mit dem Online, also in Echtzeit, sowohl aus einer Gasphase als auch aus einer Flüssigkeit simultan gemessen werden kann und das darüber hinaus prozesssicher und automatisiert betrieben werden kann.

Zusammenfassend lässt sich sagen, dass ein hoher Bedarf an einer Vorrichtung zum massenspektrometrischen Analysieren von Substanzen besteht, mit der sowohl gasförmige als auch flüssige Proben ohne Umbau dieser Vorrichtung automatisiert, insbesondere zur Prozessbeobachtung, analysiert werden können.

Es ist somit Aufgabe der vorliegenden Erfindung, insbesondere die obengenannten Nachteile zu überwinden und insbesondere ein Verfahren und eine Vorrichtung zum Durchführen einer massenspektrometrischen Analyse von in flüssigen und gasförmigen Proben vorhandenen Substanzen, bevorzugt in Echtzeit und vollautomatisiert, analysieren und bestimmen zu können. Insbesondere soll sich das vorliegende Verfahren und die vorliegende Vorrichtung dadurch auszeichnen, dass die Vorrichtung einmalig in einem Reaktionssystem verbaut wird und dann in Echtzeit mit einer Auslastung von bevorzugt mindestens 90 %, bevorzugt mindestens 95 %, die in dem Reaktionssystem vorhandenen und der Vorrichtung zuführbaren gasförmigen und flüssigen Proben vollständig automatisiert analysiert und die darin flüchtigen Substanzen bestimmt sowie bei auftretenden Fehlern oder Problemen eigenständig, bevorzugt software- oder computergestützt, diese erkennt und gegebenenfalls geeignete Sicherungsmaßnahmen dagegen ergreift. Insbesondere liegt der Erfindung daher die Aufgabe zugrunde, ein Verfahren und eine Vorrichtung zur Bestimmung flüchtiger Substanzen oder in Gasform vorliegender Substanzen zu schaffen, welche auch eine kontinuierliche Bestimmung dieser Substanzen insbesondere aus fließenden Flüssigkeiten in Echtzeit gestatten, sodass, beispielsweise in der chemischen Industrie, Regelungsvorgänge hinsichtlich der Konzentration von bestimmten Substanzen in einer Flüssigkeit und einem Gasgemisch vorgenommen werden können.

Die Aufgabe wird gelöst, indem die Gegenstände der unabhängigen Ansprüche bereitgestellt werden. Vorteilhafte Ausgestaltungen ergeben sich aus den Unteransprüchen.

Die vorliegende Erfindung betrifft insbesondere ein Verfahren zum massenspektrometrischen Analysieren von in flüssigen und gasförmigen Proben vorhandenen Substanzen, umfassend die Schritte:
a) Einbringen mindestens einer oder mehrerer Substanzen einer flüssigen, bevorzugt wässrigen, Probe in ein erstes Durchflusselement einer Vorrichtung durch Verflüchtigen der mindestens einen Substanz aus der flüssigen, bevorzugt wässrigen Probe, an einer flüssigkeitsundurchlässigen und gasdurchlässigen Membran und Einbringen mindestens einer oder mehrerer Substanzen einer gasförmigen Probe in ein zweites Durchflusselement der Vorrichtung, wobei das erste Durchflusselement von dem zweiten Durchflusselement verschieden ist, sodass nach Einbringen der mindestens einen oder der mehreren Substanzen in das erste und zweite Durchflusselement die mindestens eine Substanz gasförmig in dem jeweiligen Durchflusselement vorliegt, und
b) massenspektrometrisches Analysieren der mindestens einen oder der mehreren in Schritt a) gasförmig vorliegenden Substanzen aus der flüssigen Probe und der mindestens einen oder der mehreren in Schritt a) gasförmig vorliegenden Substanzen aus der gasförmigen Probe.

Durch das, bevorzugt ventilgesteuerte, Einbringen der mindestens einen Substanz der flüssigen Probe in ein erstes Durchflusselement und das, bevorzugt zeitlich versetzte, Einbringen der mindestens einen Substanz der gasförmigen Probe in ein zweites Durchflusselement ist es vorteilhafterweise möglich, mit einer einzigen Vorrichtung mindestens eine oder mehrere Substanzen aus sowohl flüssigen als auch gasförmigen Proben massenspektrometrisch zu analysieren oder zu bestimmen. Durch das Einbringen der mindestens einen Substanz der gasförmigen und der flüssigen Probe mittels zwei unterschiedlicher Durchflusselemente in ein und dieselbe Vorrichtung wird vorteilhafterweise erreicht, dass ein Umbau der Vorrichtung nicht mehr notwendig ist, falls die zu messenden Proben unterschiedliche Aggregatszustände aufweisen. Dadurch können in einfacher Weise "gleichzeitig" Substanzen aus gasförmigen und flüssigen Proben analysiert oder bestimmt werden. Insbesondere können diese Substanzen, zwar zeitlich versetzt, aber ohne Umbau der Vorrichtung zur massenspektrometrischen Analyse, kontinuierlich über einen langen Zeitraum analysiert oder bestimmt werden.

Insbesondere kann das erfindungsgemäße Verfahren automatisiert durchgeführt werden.

Erfindungsgemäß ist daher insbesondere vorgesehen, dass mindestens eine Substanz aus einer flüssigen Probe in ein erstes Durchflusselement und mindestens eine Substanz einer gasförmigen Probe in ein zweites Durchflusselement der erfindungsgemäß eingesetzten Vorrichtung eingebracht werden, das heißt in zwei verschiedene, also nicht dasselbe Durchflusselement, eingebracht werden. Erfindungsgemäß ist das erste Durchflusselement von dem zweiten Durchflusselement verschieden insofern, als dass das erste Durchflusselement für das Einbringen mindestens einer Substanz aus einer flüssigen Probe und das zweite Durchflusselement für das Einbringen mindestens einer Substanz aus einer gasförmigen Probe vorgesehen ist.

Die Erfindung sieht ein vorgenanntes Verfahren vor, gemäß dem in Schritt a), insbesondere ventilgesteuert, mindestens eine oder mehrere Substanzen einer flüssigen Probe in ein erstes Durchflusselement einer Vorrichtung zur massenspektrometrischen Analyse von in flüssigen und gasförmigen Proben vorhandenen Substanzen und mindestens eine oder mehrere Substanzen einer gasförmigen Probe in ein zweites Durchflusselement dieser Vorrichtung, welches von dem ersten Durchflusselement verschieden ist, eingebracht wird oder werden und wobei dies so geschieht, dass nach Einbringen der mindestens einen oder mehreren Substanzen in das erste und zweite Durchflusselement die mindestens eine oder mehrere Substanzen in dem ersten und zweiten Durchflusselement jeweils zumindest teilweise, bevorzugt vollständig, gasförmig vorliegt oder vorliegen.

Die Erfindung stellt ein Verfahren zur massenspektrometrischen Analyse unter Nutzung einer Vorrichtung zum massenspektrometrischen Analysieren von in flüssigen und gasförmigen Proben vorhandenen Substanzen bereit, wobei, insbesondere ventilgesteuert, in Schritt a) die mindestens eine oder mehrere Substanzen aus einer flüssigen, bevorzugt wässrigen, Probe verflüchtigt, das heißt in den gasförmigen Zustand überführt wird und anschließend in ein erstes Durchflusselement der Vorrichtung teilweise, bevorzugt vollständig, in gasförmigen Zustand, das heißt verflüchtigt, eingebracht wird oder werden und wobei mindestens eine oder mehrere Substanzen aus der gasförmigen Probe in ein zweites von dem ersten Durchflusselement verschiedenes Durchflusselement der Vorrichtung eingebracht wird oder werden, sodass nach Einbringen der mindestens einen oder mehreren Substanzen in das erste und zweite Durchflusselement die mindestens eine oder mehrere Substanzen zumindest teilweise, bevorzugt vollständig, gasförmig in dem jeweiligen Durchflusselement vorliegen.

Erfindungsgemäß wird demnach ein Verfahren zum massenspektrometrischen Analysieren von in flüchtigen und gasförmigen Proben vorhandenen Substanzen bereitgestellt, wobei in Schritt a) sowohl mindestens eine oder mehrere Substanzen einer flüssigen Probe in ein erstes Durchflusselement der eingesetzten Vorrichtung als auch mindestens eine oder mehrere Substanzen einer gasförmigen Probe in ein zweites Durchflusselement der Vorrichtung eingebracht werden, insbesondere zeitlich versetzt eingebracht werden, sodass nach Einbringen, bevorzugt nach dem zeitlich versetzten Einbringen, der mindestens einen oder mehreren Substanzen in das erste und zweite Durchflusselement die mindestens eine oder mehrere Substanzen zumindest teilweise, bevorzugt vollständig, gasförmig in dem jeweiligen Durchflusselement vorliegen, bevorzugt zeitlich versetzt vorliegen.

Die Erfindung sieht vor, in einem Verfahrensschritt b) die aus der flüssigen Probe stammende mindestens eine Substanz und die aus der gasförmigen Probe stammende mindestens eine Substanz getrennt voneinander massenspektrometrisch zu analysieren.

In einem Reaktionssystem, das mindestens eine zu analysierende Substanz in einer flüssigen und gasförmigen Phase enthält, kann vorteilhafterweise durch ein zeitlich versetztes Einbringen mindestens einer Substanz aus einer flüssigen Probe und mindestens einer Substanz aus einer gasförmigen Probe des Reaktionssystems der Gehalt der in flüchtige Form überführbaren Substanzen, z.B. der Gesamtgehalt zu der analysierenden Substanz, in dem vermessenen Reaktionssystem aus flüssiger und gasförmiger Phase bestimmt werden.

Das erfindungsgemäße Verfahren wird vorzugsweise mittels einer erfindungsgemäßen Vorrichtung durchgeführt, wobei diese erfindungsgemäße Vorrichtung eine Vorrichtung zur massenspektrometrischen Analyse von in flüssigen und gasförmigen Proben vorhandenen Substanzen ist, aufweisend zumindest aa) ein Massenspektrometer, bb) mindestens einen Einlass für eine flüssige Probe und cc) mindestens einen Einlass für eine gasförmige Probe, wobei dem mindestens einen Einlass für eine flüssige Probe ein erstes Durchflusselement und dem mindestens einen Einlass für eine gasförmige Probe ein zweites Durchflusselement jeweils nachgeschaltet sind, wobei das erste Durchflusselement verschieden von dem zweiten Durchflusselement ist, und wobei im Einlass für eine flüssige Probe eine hydrophobe zumindest teilweise poröse Membran angeordnet ist.

Erfindungsgemäß bevorzugt ist der mindestens eine Einlass für das Einbringen mindestens einer Substanz einer flüssigen Probe ein dem ersten Durchflusselement probenseitig vorgeschaltetes Element.

Erfindungsgemäß bevorzugt ist der mindestens eine Einlass für das Einbringen mindestens einer Substanz aus einer gasförmigen Probe ein dem zweiten Durchflusselement probenseitig vorgeschaltetes Element.

Erfindungsgemäß ist demnach vorgesehen, dass die in der flüssigen Probe zu analysierende mindestens eine Substanz in oder an einer im Einlass für die flüssige Probe vorhandenen Membraneinrichtung verflüchtigt wird. In einer besonders bevorzugten Ausführungsform kann der mindestens eine Einlass für die flüssige Probe als ein eine Membran aufweisender in situ-Sensor ausgeführt sein. Die Membraneinrichtung kann auch als Membraneinrichtung ausgebildet sein, an der die flüssige Probe vorbeigeführt wird.

Die vorliegende Erfindung stellt ein Verfahren zum massenspektrometrischen Analysieren von in einem Reaktionssystem, umfassend eine flüssige und eine gasförmige Phase, vorhandenen Substanzen zur Verfügung, umfassend die Schritte:
a) Einbringen mindestens einer Substanz einer flüssigen Probe des Reaktionssystems in ein erstes Durchflusselement einer Vorrichtung durch Verflüchtigen der mindestens einen Substanz aus der flüssigen Probe an einer flüssigkeitsundurchlässigen und gasdurchlässigen Membran und Einbringen mindestens einer Substanz einer gasförmigen Probe des Reaktionssystems in ein zweites Durchflusselement der Vorrichtung, wobei das erste Durchflusselement von dem zweiten Durchflusselement verschieden ist, sodass nach Einbringen der mindestens einen Substanz in das erste und zweite Durchflusselement die mindestens eine Substanz die mindestens eine Substanz in dem ersten und zweiten Durchflusselement gasförmig vorliegt, und
b) massenspektrometrisches Analysieren der mindestens einen in Schritt a) gasförmig vorliegenden Substanz aus der flüssigen Probe und der mindestens einen in Schritt a) gasförmig vorliegenden Substanz aus der gasförmigen Probe.

Erfindungsgemäß wird in die Vorrichtung bei Durchführung des erfindungsgemäßen Verfahrens in Schritt a) sowohl mindestens eine Substanz einer flüssigen Probe in ein erstes Durchflusselement als auch, bevorzugt zeitlich versetzt, mindestens eine Substanz einer gasförmigen Probe in ein zweites Durchflusselement eingebracht, sodass, bevorzugt zeitlich versetzt, in beiden Durchflusselementen die mindestens eine Substanz jeweils gasförmig vorliegt.

Ein erfindungsgemäßes Verfahren ermöglicht es, in einem eine gasförmige und eine flüssige Phase aufweisenden Reaktionssystem, insbesondere einem geschlossenen Reaktionssystem, z.B. die Gesamtmenge an mindestens einer flüchtigen Substanz sowohl in der flüssigen als auch der gasförmigen Phase mittels einer einzigen Vorrichtung, nämlich der erfindungsgemäßen Vorrichtung in einem einfachen und effizienten Verfahren zu bestimmen. Die erfindungsgemäße Verfahrensweise ermöglicht es, teilweise oder vollständige Stoffwechselbilanzen in den verschiedensten Reaktionssystemen, insbesondere für die verschiedensten Reaktionen, insbesondere biotechnologischen Reaktionen, bereitzustellen.

Die vorliegende Erfindung sieht darüber hinaus in einer bevorzugten Ausführungsform ein Verfahren vor, wobei mittels eines optional in der erfindungsgemäßen Vorrichtung vorhandenen Detektors zur Bestimmung nicht-flüchtiger Substanzen in einer flüssigen Phase zusätzlich in nicht-flüchtiger Form vorliegende Substanzen oder Substanzanteile bestimmt werden.

In einer besonders bevorzugten Ausführungsform wird ein vorstehend genanntes Verfahren mit den Schritten a) und b) bereitgestellt, wobei insbesondere die nachstehend beschriebenen Verfahrensschritte a), a1), a2), sowie b), insbesondere i), ii), iii) und iv), durchgeführt werden.

Die vorliegende Erfindung betrifft bevorzugt ein Verfahren, wobei in einem vor dem Schritt b) liegenden Schritt a1) die mindestens eine oder mehreren gasförmig vorliegenden Substanzen in ein drittes Durchflusselement der Vorrichtung eingebracht werden, wobei in dem dritten Durchflusselement ein Druck von 0,01 bis 0,5 mbar, bevorzugt 0,15 bis 0,2 mbar vorliegt. Bevorzugt werden die mindestens eine oder mehreren gasförmig vorliegenden Substanzen aus der gasförmigen Probe in ein erstes drittes Durchflusselement und die mindestens eine oder mehreren gasförmig vorliegenden Substanzen aus der flüssigen Probe in ein zweites drittes Durchflusselement eingebracht, wobei in dem ersten und/oder zweiten dritten Durchflusselement ein Druck von 0,01 bis 0,5 mbar, bevorzugt 0,15 bis 0,2 mbar vorliegt. Alternativ bevorzugt werden die mindestens eine oder mehreren gasförmig vorliegenden Substanzen aus der gasförmigen Probe und der flüssigen Probe in dasselbe dritte Durchflusselement eingebracht, insbesondere zeitlich versetzt und getrennt voneinander.

Die vorliegende Erfindung betrifft bevorzugt ein Verfahren, wobei in einem vor dem Schritt b) liegenden Schritt a2) die mindestens eine oder mehreren gasförmig vorliegenden Substanzen in ein viertes Durchflusselement der Vorrichtung eingebracht werden, wobei in dem vierten Durchflusselement ein Druck von 10⁻⁵ mbar oder weniger, bevorzugt 10⁻⁶ mbar oder weniger, bevorzugt 10⁻⁷ mbar oder weniger, bevorzugt 10⁻⁵ mbar bis 10⁻¹⁰ mbar, vorliegt.

Bevorzugt werden die mindestens eine oder mehreren gasförmig vorliegenden Substanzen zunächst in das dritte und anschließend in das vierte Durchflusselement eingebracht. Bevorzugt werden die mindestens eine oder mehreren gasförmig vorliegenden Substanzen der gasförmigen Probe zunächst in ein erstes drittes Durchflusselement und anschließend in ein erstes viertes Durchflusselement eingebracht. Bevorzugt werden die mindestens eine oder mehreren gasförmig vorliegenden Substanzen der flüssigen Probe zunächst in ein zweites drittes Durchflusselement und anschließend in ein zweites viertes Durchflusselement eingebracht. Alternativ bevorzugt werden die mindestens eine oder mehreren gasförmig vorliegenden Substanzen in dem ersten dritten und dem zweiten dritten Durchflusselement in dasselbe vierte Durchflusselement eingebracht. Bevorzugt liegt in dem ersten und/oder zweiten vierten Durchflusselement ein Druck von 10⁻⁵ mbar oder weniger, bevorzugt 10⁻⁶ mbar oder weniger, bevorzugt 10⁻⁷ mbar oder weniger, bevorzugt 10⁻⁵ mbar bis 10⁻¹⁰ mbar, vor.

Die Erfindung betrifft bevorzugt ein Verfahren, wobei das massenspektrometrische Analysieren gemäß Schritt b) die folgenden Schritte umfasst:
i) Ionisieren der mindestens einen oder mehreren gasförmig vorliegenden Substanzen aus der flüssigen Probe und der mindestens einen oder mehreren gasförmig vorliegenden Substanzen aus der gasförmigen Probe,
ii) Beschleunigen der mindestens einen oder mehreren in Schritt i) ionisierten Substanzen,
iii) Selektieren der mindestens einen oder mehreren in Schritt ii) beschleunigten Substanzen, und
iv) Detektieren der mindestens einen oder mehreren in Schritt iii) selektierten Substanzen.

Bevorzugt umfasst das massenspektrometrische Analysieren gemäß Schritt b) ein Ionisieren der mindestens einen oder mehreren gasförmig vorliegenden Substanzen und ein anschließendes Detektieren der mindestens einen oder mehreren ionisierten Substanzen.

Bevorzugt wird ein Verfahren, das folgende Schritte umfasst:
a) Einbringen mindestens einer oder mehrerer Substanzen einer flüssigen Probe in ein erstes Durchflusselement einer Vorrichtung durch Verflüchtigen der mindestens einen oder mehreren Substanzen aus der flüssigen Probe an einer flüssigkeitsundurchlässigen und gasdurchlässigen Membran und Einbringen mindestens einer oder mehrerer Substanzen einer gasförmigen Probe in ein zweites Durchflusselement der Vorrichtung, wobei das erste Durchflusselement von dem zweiten Durchflusselement verschieden ist, sodass nach Einbringen der mindestens einen oder mehreren Substanzen in das erste und zweite Durchflusselement die mindestens eine oder mehreren Substanzen zumindest teilweise, bevorzugt vollständig, gasförmig vorliegen,
a1) zumindest teilweises, bevorzugt teilweises oder vollständiges, Einbringen der mindestens einen oder mehreren in dem ersten und zweiten Durchflusselement gasförmig vorliegenden Substanzen in ein drittes Durchflusselement der Vorrichtung, wobei in dem dritten Durchflusselement ein Druck von 0,01 bis 0,5 mbar, bevorzugt 0,15 bis 0,20 mbar, vorliegt,
a2) zumindest teilweises, bevorzugt teilweises oder vollständiges, Einbringen der mindestens einen oder mehreren in dem dritten Durchflusselement gasförmig vorliegenden Substanzen in ein viertes Durchflusselement der Vorrichtung, wobei in dem vierten Durchflusselement ein Druck von 10⁻⁵ mbar oder weniger, bevorzugt 10⁻⁶ mbar oder weniger, bevorzugt 10⁻⁷ mbar oder weniger, bevorzugt 10⁻⁵ mbar bis 10⁻¹⁰ mbar, vorliegt und
b) zumindest teilweises, bevorzugt teilweises oder vollständiges, massenspektrometrisches Analysieren der mindestens einen oder mehreren in dem vierten Durchflusselement gasförmig vorliegenden Substanzen, wobei das massenspektrometrische Analysieren bevorzugt die folgenden Schritte umfasst:
   i) zumindest teilweises, bevorzugt teilweises oder vollständiges, Ionisieren der mindestens einen oder mehreren in dem vierten Durchflusselement gasförmig vorliegenden Substanzen,
   ii) zumindest teilweises, bevorzugt teilweises oder vollständiges, Beschleunigen der mindestens einen oder mehreren in Schritt i) ionisierten Substanzen,
   iii) zumindest teilweises, bevorzugt teilweises oder vollständiges, Selektieren der mindestens einen oder mehreren in Schritt ii) beschleunigten Substanzen, und
   iv) zumindest teilweises, bevorzugt teilweises oder vollständiges, Detektieren der mindestens einen oder mehreren in Schritt iii) selektierten Substanzen.

Bevorzugt ist das erste, das zweite, das dritte, bevorzugt das erste und/oder zweite dritte, und/oder das vierte, bevorzugt das erste und/oder zweite vierte, Durchflusselement eine Leitung, bevorzugt ein Rohr oder ein Schlauch, bevorzugt ein Metallrohr oder Metallwellschlauch, bevorzugt ein Edelstahlrohr oder Edelstahlwellschlauch.

In besonders bevorzugter Ausführungsform können das erste, das zweite, das dritte und das vierte Durchflusselement, insbesondere das erste und das zweite Durchflusselement, baugleich ausgestaltet sein.

Das erfindungsgemäße Verfahren und die erfindungsgemäße Vorrichtung sind insbesondere vorteilhafterweise einsetzbar für die quantitative und qualitative Bestimmung von flüchtigen Substanzen in einem Reaktionssystem, welches Reaktionssystem aus einer flüssigen und einer gasförmigen Phase aufgebaut ist, insbesondere eine Flüssigkeit und ein Gas oder ein Gasgemisch aufweist. Das erfindungsgemäße Verfahren und die erfindungsgemäße Vorrichtung ermöglichen es, die flüchtigen Substanzen simultan oder zeitlich versetzt sowohl in der flüssigen als auch in der gasförmigen Phase eines solchen Reaktionssystems zu bestimmen, sodass vorteilhaft beispielsweise Edukt- und Produktkonzentrationen in Reaktionssystemen erfasst werden können, in denen im Rahmen einer oder mehrerer biochemischer Reaktionen Aggregatszustandsänderungen auftreten. Edukt- und Produktkonzentrationen können so simultan oder nahezu simultan erfasst werden, umso ein möglichst exaktes Bild einer zu einem bestimmten Zeitpunkt vorliegenden Umsetzung zu erhalten ohne oder ohne großen zeitlichen Versatz zwischen Messung des Eduktes und des Produktes. Dies ermöglicht eine direkte Erfassung und Überwachung der jeweiligen Reaktion. Die erfindungsgemäße Verfahrensweise und Vorrichtung ermöglichen eine derartige Vorgehensweise vorteilhafterweise mittels einer einzigen Vorrichtung und eines damit durchgeführten Verfahrens.

Die vorliegende Erfindung stellt daher auch Verfahren zum massenspektrometrischen Analysieren von in einem aus mindestens einer flüssigen und mindestens einer gasförmigen Phase aufgebauten Reaktionssystem enthaltenden Substanzen zur Verfügung, wobei ein Verfahren zum massenspektrometrischen Analysieren von in flüssigen und gasförmigen Proben vorhandenen Substanzen gemäß der vorliegenden Erfindung, insbesondere umfassend die Schritte a) und b), durchgeführt wird, um die Substanzen zu bestimmen, bevorzugt umfassend die Schritte a), a1) und a2) und/oder in Schritt b) die Schritte i), ii), iii) und iv).

In besonders bevorzugter Ausführungsform ist ein solches Reaktionssystem ein mindestens ein flüssiges Reaktionsmedium und mindestens eine Gasphase aufweisender Bioreaktor, insbesondere für die Fermentation, den Bioabbau von Produkten, die Umsetzung von Substanzen oder die Herstellung von Substanzen.

In besonders bevorzugter Ausführungsform kann mittels dieses Verfahrens sowohl die Konzentration der Ausgangsstoffe als auch der Endprodukte mittels einer einzigen Vorrichtung effizient bestimmt werden.

In bevorzugter Ausführungsform kann ein eine flüssige und gasförmige Phase aufweisendes Reaktionssystem im Bereich der Biotechnologie, der chemischen Industrie, der Petrochemie, Pharmazie, Medizintechnologie, und Lebensmittelindustrie eingesetzt werden.

Unter dem Begriff "und/oder" wird in Zusammenhang mit der vorliegenden Verbindung verstanden, dass die Mitglieder einer Gruppe, welche durch den Begriff "und/oder" miteinander verbunden sind, alternativ zueinander, teilweise kumulativ oder vollständig kumulativ offenbart sind. Dies bedeutet beispielsweise für den Ausdruck "A, B, und/oder C", dass folgender Offenbarungsgehalt darunter zu verstehen ist: A oder B oder C oder (A und B) oder (A und C) oder (B und C) oder (A und B und C).

Bevorzugt ist das erste, das zweite und das dritte Durchflusselement durch eine erste Ventileinrichtung, bevorzugt unmittelbar, miteinander verbunden. Bevorzugt ist das dritte Durchflusselement über eine zweite Ventileinrichtung, bevorzugt unmittelbar, mit dem vierten Durchflusselement verbunden. Bevorzugt verbindet die erste Ventileinrichtung in einer ersten Stellung das erste Durchflusselement mit dem dritten Durchflusselement. In einer zweiten Stellung verbindet die erste Ventileinrichtung das zweite Durchflusselement mit dem dritten Durchflusselement. In einer dritten Stellung besteht zwischen dem ersten und dem zweiten Durchflusselement sowie dem dritten Durchflusselement kein Fluidkontakt. Bevorzugt verbindet die zweite Ventileinrichtung in einer ersten Stellung das dritte Durchflusselement mit dem vierten Durchflusselement. In einer zweiten Stellung besteht zwischen dem dritten Durchflusselement und dem vierten Durchflusselement kein Fluidkontakt.

In einer alternativen Ausführungsform ist das erste Durchflusselement mit dem dritten Durchflusselement durch eine erste Ventileinrichtung, bevorzugt unmittelbar, miteinander verbunden. Zusätzlich ist das zweite Durchflusselement mit dem dritten Durchflusselement durch eine zweite Ventileinrichtung, bevorzugt unmittelbar, miteinander verbunden. Bevorzugt ist das dritte Durchflusselement über eine dritte Ventileinrichtung, bevorzugt unmittelbar, mit dem vierten Durchflusselement verbunden. Bevorzugt verbindet die erste Ventileinrichtung in einer ersten Stellung das erste Durchflusselement mit dem dritten Durchflusselement. In einer zweiten Stellung besteht zwischen dem ersten und dem dritten Durchflusselement kein Fluidkontakt. Bevorzugt verbindet die zweite Ventileinrichtung in einer ersten Stellung das zweite Durchflusselement mit dem dritten Durchflusselement. In einer zweiten Stellung besteht zwischen dem zweiten und dem dritten Durchflusselement kein Fluidkontakt. Bevorzugt verbindet die dritte Ventileinrichtung in einer ersten Stellung das dritte Durchflusselement mit dem vierten Durchflusselement. In einer zweiten Stellung besteht zwischen dem dritten und dem vierten Durchflusselement kein Fluidkontakt.

In einer alternativen Ausführungsform ist das erste Durchflusselement mit dem ersten dritten Durchflusselement durch eine erste Ventileinrichtung, bevorzugt unmittelbar, miteinander verbunden. Zusätzlich ist das zweite Durchflusselement mit dem zweiten dritten Durchflusselement durch eine zweite Ventileinrichtung, bevorzugt unmittelbar, miteinander verbunden. Zusätzlich ist das erste dritte, das zweite dritte und das vierte Durchflusselement durch eine dritte Ventileinrichtung, bevorzugt unmittelbar, miteinander verbunden. Bevorzugt verbindet dabei die erste Ventileinrichtung in einer ersten Stellung das erste Durchflusselement mit dem ersten dritten Durchflusselement. In einer zweiten Stellung besteht zwischen dem ersten Durchflusselement und dem ersten dritten Durchflusselement kein Fluidkontakt. Bevorzugt verbindet die zweite Ventileinrichtung in einer ersten Stellung das zweite Durchflusselement mit dem zweiten dritten Durchflusselement. In einer zweiten Stellung besteht zwischen dem zweiten Durchflusselement und dem zweiten dritten Durchflusselement kein Fluidkontakt. Bevorzugt verbindet die dritte Ventileinrichtung in einer ersten Stellung das erste dritte Durchflusselement mit dem vierten Durchflusselement. Bevorzugt verbindet die dritte Ventileinrichtung in einer zweiten Stellung das zweite dritte Durchflusselement mit dem vierten Durchflusselement. In einer dritten Stellung besteht zwischen dem ersten dritten Durchflusselement, dem zweiten dritten Durchflusselement und dem vierten Durchflusselement kein Fluidkontakt.

In einer alternativen Ausführungsform ist das erste Durchflusselement mit dem ersten dritten Durchflusselement durch eine erste Ventileinrichtung, bevorzugt unmittelbar, miteinander verbunden. Zusätzlich ist das zweite Durchflusselement mit dem zweiten dritten Durchflusselement durch eine zweite Ventileinrichtung, bevorzugt unmittelbar, miteinander verbunden. Zusätzlich ist das erste dritte Durchflusselement mit dem ersten vierten Durchflusselement durch eine dritte Ventileinrichtung, bevorzugt unmittelbar, miteinander verbunden. Zusätzlich ist das zweite dritte Durchflusselement mit dem zweiten vierten Durchflusselement durch eine vierte Ventileinrichtung, bevorzugt unmittelbar, miteinander verbunden. Bevorzugt verbindet dabei die erste Ventileinrichtung in einer ersten Stellung das erste Durchflusselement mit dem ersten dritten Durchflusselement. In einer zweiten Stellung besteht zwischen dem ersten Durchflusselement und dem ersten dritten Durchflusselement kein Fluidkontakt. Bevorzugt verbindet die zweite Ventileinrichtung in einer ersten Stellung das zweite Durchflusselement mit dem zweiten dritten Durchflusselement. In einer zweiten Stellung besteht zwischen dem zweiten Durchflusselement und dem zweiten dritten Durchflusselement kein Fluidkontakt. Bevorzugt verbindet die dritte Ventileinrichtung in einer ersten Stellung das erste dritte Durchflusselement mit dem ersten vierten Durchflusselement. In einer zweiten Stellung besteht zwischen dem ersten dritten Durchflusselement und dem ersten vierten Durchflusselement kein Fluidkontakt. Bevorzugt verbindet die vierte Ventileinrichtung in einer ersten Stellung das zweite dritte Durchflusselement mit dem zweiten vierten Durchflusselement. In einer zweiten Stellung besteht zwischen dem zweiten dritten Durchflusselement und dem zweiten vierten Durchflusselement kein Fluidkontakt.

Bevorzugt weist jeder Einlass für eine gasförmige Probe oder eine flüssige Probe ein drittes und viertes Durchlaufelement auf, wobei erst unmittelbar vor dem Massenspektrometer die unterschiedlichen Durchflusselementstränge über eine Ventileinrichtung zusammengeführt werden.

Bevorzugt ist jedem Einlass für eine flüssige Probe ein erstes Durchflusselement, ein drittes und ein viertes Durchflusselement und jedem Einlass für eine gasförmige Probe ein zweites Durchflusselement, ein drittes und ein viertes Durchflusselement zugeordnet, wobei erst unmittelbar vor dem Massenspektrometer die unterschiedlichen Durchflusselementstränge über eine Ventileinrichtung zusammengeführt werden.

Durch die erste, zweite, dritte und/oder vierte Ventileinrichtung ist es insbesondere möglich, den Massenstrom an gasförmigen Substanzen in das oder die dritte(n) und/oder vierte(n) Durchflusselement(e) gezielt zu steuern.

Bevorzugt weist die erste, zweite, dritte und/oder vierte Ventileinrichtung mindestens ein, bevorzugt mindestens zwei, bevorzugt genau ein, bevorzugt genau zwei Ventile auf. Bevorzugt ist das mindestens eine Ventil ein Schaltventil, Trommelventil oder ein Proportionalventil. Das mindestens eine Ventil ist bevorzugt als elektrisches oder pneumatisches Ventil ausgebildet.

Bevorzugt sind die Ventile, bevorzugt alle Ventile, ansteuerbar, bevorzugt über ein Software-basiertes Steuerungsprogramm oder eine speicherprogrammierbare Steuerung.

Bevorzugt wird in das erste Durchflusselement zum Kalibrieren des Massenspektrometers anstatt einer flüssigen Probe eine Kalibrierlösung eingebracht. Bevorzugt wird zum Kalibrieren des Massenspektrometers anstelle einer gasförmigen Probe, bevorzugt ventilgesteuert, in das zweite Durchflusselement ein Kalibriergas oder Kalibriergemisch eingebracht.

Die vorliegende Erfindung betrifft bevorzugt ein Verfahren, wobei das erste, das zweite, das dritte und/oder das vierte Durchflusselement auf eine Temperatur von 60 bis 80 °C, bevorzugt 70 bis 75 °C, erhitzt wird. Das Erhitzen der Durchflusselemente erfolgt bevorzugt durch ein Heizelement. Bevorzugt ist dem ersten, dem zweiten, dem dritten und/oder dem viertem Durchflusselement jeweils ein Heizelement zugeordnet. Bevorzugt liegt in dem ersten, dem zweiten, dem dritten und/oder dem viertem Durchflusselement die gleiche Temperatur vor.

Der Begriff "das dritte Durchflusselement" oder "das vierte Durchflusselement" umfasst ein oder mehrere Durchflusselemente derselben Art. Dementsprechend umfassen diese Begriffe auch das erste dritte/vierte und das zweite dritte/vierte Durchflusselement, falls in der Vorrichtung vorhanden.

Durch das Erhitzen eines oder mehrerer Durchflusselemente wird das Abscheiden, bevorzugt das Kondensieren, oder Adsorbieren der mindestens einen oder mehreren gasförmig vorliegenden Stoffe oder Substanzen an der Innenseite des Durchflusselementes, bevorzugt der Leitung, bevorzugt des Rohres oder des Schlauches, bevorzugt des Metallrohres oder des Metallwellschlauches, bevorzugt des Edelstahlrohres oder des Edelstahlwellschlauches, verhindert.

Insbesondere wird durch die erste, zweite, dritte und/oder vierte Ventileinrichtung verhindert, dass eine zu große Menge der gasförmigen oder flüssigen Probe und/oder eines Kalibriermediums in die Durchflusselemente, bevorzugt in das dritte und/oder vierte Durchflusselement, oder in das Massenspektrometer gelangen. Somit wird eine Beschädigung insbesondere des Massenspektrometers minimiert, bevorzugt verhindert.

Die vorliegende Erfindung betrifft bevorzugt ein Verfahren, wobei das Einbringen der mindestens einen oder mehreren Substanzen der flüssigen und gasförmigen Probe in das erste und zweite Durchflusselement derart gesteuert wird, dass in Schritt a1) und/oder a2) gleiche Mengen der mindestens einen oder mehreren gasförmigen Substanzen in dem dritten Durchflusselement vorliegen. Erfindungsgemäß wird unter dem Begriff "gleiche Mengen" verstanden, dass sich, bevorzugt über die Steuerung der Ventileinrichtung, der Massenstrom aus dem ersten Durchflusselement in das dritte Durchflusselement und der Massenstrom aus dem zweiten Durchflusselement in das dritte Durchflusselement maximal um ±10 %, bevorzugt maximal um ±5 %, bevorzugt maximal um ±1 % unterscheiden. Bevorzugt wird unter dem Begriff "Massenstrom" die in das dritte Durchflusselement eingeleitete Masse der mindestens einen oder mehreren Substanzen über eine bestimmte Zeit verstanden. Durch diese spezielle Verfahrensführung, nämlich durch das Einbringen gleicher Mengen der mindestens einen oder mehreren gasförmigen Substanzen in das dritte Durchflusselement, ist es insbesondere möglich, in einer vollautomatisierten Weise zwischen dem ersten und dem zweiten Durchflusselement, also zwischen dem Einbringen der mindestens einen oder mehreren Substanzen einer gasförmigen oder einer flüssigen Probe hin und her zu schalten. Insbesondere ist es so möglich, die Vorrichtung zum massenspektrometrischen Analysieren mit einem einzigen Kalibriermedium zu kalibrieren und anschließend über das erste und das zweite Durchflusselement unterschiedliche Proben einzuleiten.

Bevorzugt werden über das erste Durchflusselement mindestens eine oder mehrere Substanzen von mindestens zwei, bevorzugt mindestens fünf, bevorzugt mindestens zehn unterschiedlichen flüssigen Proben über jeweils unterschiedliche Einlässe, bevorzugt zeitlich versetzt, eingebracht.

Bevorzugt werden über das erste Durchflusselement mindestens eine, bevorzugt mindestens zwei, bevorzugt mindestens fünf, bevorzugt mindestens zehn, bevorzugt maximal dreißig, bevorzugt maximal zwanzig unterschiedliche Substanzen einer flüssigen Probe eingebracht.

Bevorzugt werden über das zweite Durchflusselement mindestens eine oder mehrere Substanzen von mindestens zwei, bevorzugt mindestens fünf, bevorzugt mindestens zehn unterschiedlichen gasförmigen Proben über jeweils unterschiedliche Einlässe, bevorzugt zeitlich versetzt, eingebracht.

Bevorzugt werden über das zweite Durchflusselement mindestens eine, bevorzugt mindestens zwei, bevorzugt mindestens fünf, bevorzugt mindestens zehn, bevorzugt maximal dreißig, bevorzugt maximal zwanzig unterschiedliche Substanzen einer gasförmigen Probe eingebracht.

Die Erfindung betrifft bevorzugt ein Verfahren, wobei der Druck in dem vierten Durchflusselement detektiert wird. Bevorzugt wird der Druck in dem ersten, dem zweiten, dem dritten und/oder dem vierten Durchflusselement detektiert. Bevorzugt wird der Druck sowohl in dem dritten als auch in dem vierten Durchflusselement detektiert. Diese Detektion erfolgt bevorzugt durch jeweils eine Detektionseinheit. Bevorzugt ist diese Detektionseinheit mit einer Steuerungseinheit verbunden. Bevorzugt weist die Steuereinheit ein Software-basiertes Steuerungsprogramm oder eine speicherprogrammierbare Steuerung auf.

Die vorliegende Erfindung betrifft bevorzugt ein Verfahren, wobei bei einem detektierten Druckanstieg in dem vierten Durchflusselement eine zwischen dem dritten und vierten Durchflusselement angeordnete ansteuerbare Ventileinrichtung geschlossen wird, sodass zwischen drittem und viertem Durchflusselement kein Fluidkontakt besteht. Bevorzugt wird bei einem detektierten Druckanstieg in dem dritten Durchflusselement eine zwischen dem dritten und vierten Durchflusselement angeordnete ansteuerbare Ventileinrichtung geschlossen, sodass zwischen drittem und viertem Durchflusselement kein Fluidkontakt besteht.

Alternativ oder zusätzlich wird bei einem detektierten Druckanstieg in dem dritten Durchflusselement bevorzugt eine vor dem dritten Durchflusselement - in Strömungsrichtung der gasförmigen Substanzen - angeordnete ansteuerbare Ventileinrichtung geschlossen. Bevorzugt werden bei einem detektierten Druckanstieg in dem und/oder vierten Durchflusselement alle in der Vorrichtung vorhandenen Ventileinrichtungen geschlossen, sodass zwischen den mit diesen Ventileinrichtungen, bevorzugt unmittelbar, verbundenen Durchflusselementen kein Fluidkontakt besteht. Durch das Schließen des mindestens einen Ventils wird bevorzugt verhindert, dass eine zu große Menge an gasförmigen Substanzen, aber auch eine zu große Menge einer gasförmigen oder flüssigen Probe in die Messeinrichtung, das heißt in das zum Analysieren der mindestens einen oder mehreren gasförmigen Substanzen verwendete Massenspektrometer eindringen können.

Bevorzugt strömen die mindestens eine oder mehreren gasförmigen Substanzen aus dem vierten Durchflusselement, bevorzugt unmittelbar, in das Massenspektrometer, also in die zur Durchführung des Schritts i) ausgebildete und vorgesehene Ionisierungseinrichtung des Massenspektrometers.

Bevorzugt besteht das vorliegende Verfahren aus den vorgenannten Verfahrensschritten, bevorzugt aus den Verfahrensschritten a), a1), a2), b), i), ii), iii) und iv). Bevorzugt erfolgt keine Fraktionierung der mindestens einen oder mehreren in Schritt a) gasförmig vorliegenden Substanzen, bevor sie in Schritt b) massenspektrometrisch analysiert werden. Bevorzugt ist das erfindungsgemäße oder erfindungsgemäß bevorzugte Verfahren frei von einer chromatographischen, insbesondere gaschromatographischen, Fraktionierung, bevorzugt der mindestens einen oder mehreren gasförmig vorliegenden Substanzen.

Bevorzugt erfolgt das massenspektrometrische Analysieren von in Gasen oder Gasgemischen oder Flüssigkeiten ablaufenden Reaktionen.

Bevorzugt werden mit dem vorliegenden Verfahren Ionenströme der einen oder mehreren gasförmigen Substanzen gemessen, wodurch die jeweiligen Konzentrationen dieser Substanzen berechnet werden können.

Zur Erzeugung des in dem dritten und/oder vierten Durchflusselement vorliegenden Vakuums wird jeweils eine Pumpe verwendet, die jeweils zur Erzeugung eines entsprechenden Druckes ausgelegt ist.

Gemäß der vorliegenden Erfindung wird die flüssige Probe zum Einbringen in das erste Durchflusselement an einer flüssigkeitsundurchlässigen, aber gasdurchlässigen, bevorzugt in einer Membraneinrichtung, auch als Membranmodul bezeichnet, vorliegenden, Membran derart vorbeigeführt, dass unter den während des Verfahrens vorherrschenden Bedingungen mindestens eine oder mehrere Substanzen verflüchtigt, also in die Gasphase gebracht, werden können. Entsprechende Substanzen treten durch die Membran hindurch und werden dadurch in das erste Durchflusselement eingebracht. Bevorzugt ist vorgesehen, dass die Flüssigkeit im Bereich der Membran auf einer konstanten Temperatur, bevorzugt auf einer Temperatur zwischen 10 bis 30 °C, bevorzugt 25 °C gehalten wird.

Bevorzugt kann die Temperatur für die jeweilige Anwendung variiert werden. Bevorzugt bleibt die Temperatur während der, bevorzugt gesamten, Messdauer konstant. Bevorzugt wird bei der gleichen Temperatur kalibriert.

Bevorzugt erfolgt das Einbringen der mindestens einen oder mehreren Substanzen der flüssigen Probe in das erste Durchflusselement und das Einbringen der mindestens einen oder mehreren Substanzen der gasförmigen Probe in das zweite Durchflusselement derart, dass das erste Durchflusselement oder das zweite Durchflusselement mit dem dritten Durchflusselement über die Ventileinrichtung fluidisch, bevorzugt unmittelbar, verbunden sind, das heißt also aufgrund des in dem dritten Durchflusselements vorliegenden Unterdrucks eine gewisse Sogwirkung, aber auch Unterdruck in dem ersten und/oder zweiten Durchflusselement, entsteht.

In einer bevorzugten Ausführungsform der vorliegenden Erfindung ist vorgesehen, dass die an der Membran vorbeigeführte flüssige Probe im Ab- oder Nebenzweig zu der zu untersuchenden Flüssigkeitsströmung geführt wird. In einer bevorzugten Ausführungsform ist die Membraneinrichtung zum Einbringen flüchtiger Substanzen aus einer flüssigen Probe derart aufgebaut, dass es als Bypass fungiert, wobei die flüssige Probe aus einem Behälter oder aus einer Leitung mittels einer pulsationsarmen Pumpe entnommen und wahlweise wieder zurückgeführt wird.

Die Membran ist bevorzugt durch eine poröse Scheibe gestützt. Die Membran ist mittels eines Polytetrafluorethylen ummantelten Dichtrings in einem Membrangehäuse abgedichtet.

Zum Konstanthalten des Vakuums in dem dritten und/oder vierten Durchflusselement, werden die Vakuumdetektionsvorrichtungen, welche dem dritten und/oder dem vierten Durchflusselement zugeordnet sind, mit den diesen Durchflusselementen zugeordneten Vakuumpumpen über Regelkreise, insbesondere gesteuert über eine Steuerungseinheit, verbunden.

In einer besonders bevorzugten Ausführungsform wird ein Verfahren zum massenspektrometrischen Analysieren von in flüssigen und gasförmigen Proben vorhandenen Substanzen gemäß des vorstehend offenbarten Verfahrens bereitgestellt, umfassend zusätzlich einen Schritt des Bestimmens von in einer flüssigen Probe vorhandenen nicht-flüchtigen Substanzen. In besonders bevorzugter Ausführungsform erfolgt ein derartiges Bestimmen mittels einer erfindungsgemäßen Vorrichtung, die einen Detektor für die Bestimmung von in einer flüssigen Probe vorhandenen nicht-flüchtigen Substanzen aufweist.

Die vorliegende Erfindung betrifft auch eine Vorrichtung zum massenspektrometrischen Analysieren von in flüssigen und gasförmigen Proben vorhandenen Substanzen, aufweisend
aa) ein Massenspektrometer,
bb) mindestens einen Einlass für eine flüssige Probe und
cc) mindestens einen Einlass für eine gasförmige Probe,
wobei dem mindestens einen Einlass für eine flüssige Probe ein erstes Durchflusselement und dem mindestens einen Einlass für eine gasförmige Probe ein zweites Durchflusselement jeweils nachgeschaltet sind, wobei das erste Durchflusselement verschieden von dem zweiten Durchflusselement ist, und wobei im Einlass für eine flüssige Probe eine hydrophobe zumindest teilweise poröse Membran angeordnet ist.

Bevorzugt ist der mindestens eine Einlass für eine flüssige Probe verschieden von dem mindestens einen Einlass für eine gasförmige Probe.

Die erfindungsgemäße Vorrichtung weist bevorzugt die vorstehend beschriebenen Durchflusselemente, insbesondere ein dem mindestens einen Einlass für eine flüssige Probe nachgeschaltetes erstes Durchflusselement und ein dem mindestens einen Einlass für eine gasförmige Probe nachgeschaltetes zweites Durchflusselement, sowie optional ein drittes und/oder viertes Durchflusselement auf, wobei das erste Durchflusselement verschieden von dem zweiten Durchflusselement ist.

Die vorliegende Erfindung betrifft bevorzugt eine Vorrichtung, wobei die Vorrichtung eingerichtet ist zur Durchführung eines erfindungsgemäßen oder erfindungsgemäß bevorzugten Verfahrens.

Bevorzugt wird das erfindungsgemäße oder erfindungsgemäß bevorzugte Verfahren unter Verwendung einer erfindungsgemäßen oder erfindungsgemäß bevorzugten Vorrichtung durchgeführt.

Insbesondere zeichnet sich die vorliegende erfindungsgemäße Vorrichtung dadurch aus, dass sie eine sehr kompakte und eine totvolumenarme Vorrichtung ist.

Die vorliegende Erfindung betrifft eine Vorrichtung, wobei der mindestens eine Einlass für eine flüssige Probe eine hydrophobe, zumindest teilweise poröse Membran aufweist. Diese Membran befindet sich bevorzugt in einer Membraneinrichtung.

Die Membran ist eine hydrophobe Membran. Die Membran weist bevorzugt eine Vielzahl an Poren auf, wobei der durchschnittliche Porenradius bevorzugt einen Wert von 0,001 bis 0,1 µm, bevorzugt 0,01 bis 0,05 µm aufweist. Die Membran weist bevorzugt eine Dicke von 1 bis 100 µm, bevorzugt 10 bis 80 µm, bevorzugt 40 bis 60 µm auf. Die Membran weist bevorzugt eine Porosität von 40 bis 80 %, bevorzugt von 50 bis 70 % auf. Die Membran ist bevorzugt eine Pervaporationsmembran, bevorzugt eine Polytetrafluorethylen (PTFE)-Membran oder eine Silikonmembran, bevorzugt eine Polydimethylsiloxan (PDMS)-Membran, bevorzugt eine Polytetrafluorethylen (PTFE)-Membran. Alternativ können auch andere spezifische Membranen eingesetzt werden, die für bestimmte Stoffe selektiv sind, um so eine bessere Auflösung der Messung zu erhalten, falls erforderlich. Als Membran werden bevorzugt auch dichte Pervaporationsmembranen, bevorzugt eine Silikonmembran, bevorzugt eine PDMS-Membran verwendet.

Bevorzugt wird eine PTFE-Membran als Einlass für mindestens eine verflüchtigbare Substanz aus einer flüssigen Probe in einem Fermentationsprozess oder Zellkulturprozess verwendet, insbesondere als integraler Bestandteil eines Fermentationsprozess- oder Zellkulturprozess-Behälters, bevorzugt eines entsprechenden Einweg-Behälters.

Die Erfindung betrifft bevorzugt eine Vorrichtung, die zusätzlich mindestens einen Einlass für ein Kalibriermedium aufweist.

Die vorliegende Erfindung betrifft bevorzugt eine Vorrichtung, die mindestens eine Ventileinrichtung, welche zwischen den Einlässen und dem Massenspektrometer angeordnet ist.

Bevorzugt ist die mindestens eine Ventileinrichtung räumlich und/oder funktional zwischen den Einlässen und dem Massenspektrometer angeordnet.

Die vorliegende Erfindung betrifft bevorzugt eine Vorrichtung, wobei der mindestens eine Einlass für eine flüssige Probe als in situ-Sensor ausgebildet ist.

Besonders bevorzugt weist die vorliegende Vorrichtung eine als in situ-Sensor ausgebildete Membraneinrichtung auf. Dieser in situ-Sensor dient insbesondere dazu, direkt in einem flüssigen Reaktionsmedium oder in einem flüssigen Medium die verflüchtigbaren Substanzen aus diesem Medium zu extrahieren, sodass diese Substanzen in das erste Durchflusselement eingebracht werden können. Insbesondere kann dieser in situ-Sensor in der Biotechnologie verwendet werden, insbesondere in Form eines Ingoldstutzens. Er gilt als Standardanschlussverbindung für den Einbau in Prozessbehältern, insbesondere in Reaktoren, Rohrleitungen und/oder anderen Anlagenkomponenten.

Bevorzugt weist der in situ-Sensor ein zylindrisches Innenteil mit Außengewinde und eine Hülse mit Innengewinde auf, bevorzugt besteht er daraus, wobei das zylindrische Innenteil in die Hülse eingeschraubt ist, wobei das Innengewinde und das Außengewinde zusammenwirken. Das Innenteil weist zudem eine sich in Längsrichtung erstreckende Leitung, bevorzugt Bohrung, auf, wodurch eine Fluidverbindung zwischen dem flüssigen Medium oder Probe und dem Einlass der Vorrichtung für die flüssigen Proben vorliegt. Insbesondere ist diese Leitung elektropoliert. Durch diese Elektropolitur wird vorteilhafterweise die Adsorption, das heißt das Kondensieren der durch die Membran zugeführten gasförmig vorliegenden Stoffe an der Oberfläche dieser Leitung vermieden. Bevorzugt weist der in situ-Sensor ein Heizelement auf, sodass der gesamte Sensor beheizbar ist und auch während der Verwendung in dem erfindungsgemäß oder erfindungsgemäß bevorzugten Verfahrens beheizt wird. Die Membran wird mithilfe einer passenden Dichtung, bevorzugt O-Ringdichtung, zwischen Innenteil und der Hülse eingespannt, bevorzugt durch Festschrauben der Hülse. Die Hülse weist an diesem Ende eine Öffnung, bevorzugt Bohrung, auf, sodass die flüssige Probe in ausreichender Menge bei bestimmungsgemäßer Verwendung die Membran benetzen kann. Dementsprechend ist der einzig vorgesehene Weg der Flüssigkeit, d.h der flüssigen Probe, in das erste Durchflusselement bestimmungsgemäß allein über die Membran möglich. Diese Membran ist bevorzugt so in dem in situ-Sensor eingesetzt, dass sich eine, bevorzugt leichte, Wölbung nach außen, also in Richtung der Flüssigkeit oder Probe ergibt. Durch diese bevorzugte Wölbung wird die Kontaktfläche der Membran mit der, bevorzugt vorbeiströmenden, Flüssigkeit vergrößert, damit die Verflüchtigung der in dem Medium vorliegenden flüchtigen Substanzen verbessert und/oder die Biofilmbildung auf der Membran in einem Fermenter, insbesondere aufgrund der besseren Angriffsfläche der bevorzugt vorbeiströmenden Flüssigkeit, verringert. Zur Verhinderung der Biofilmbildung ist bevorzugt alternativ oder zusätzlich ein Wischelement vorgesehen. Bevorzugt wird diese Membran durch eine Scheibe, bevorzugt eine Sinterscheibe, gestützt, bevorzugt entgegen der Wölbung. Mittels einer ebenfalls vorhandenen Überwurfmutter kann der in situ-Sensor bevorzugt an einem Reaktionsgefäß, bevorzugt an einem Fermenter befestigt werden und schließt bevorzugt über eine an der Hülse außen anliegenden Ringdichtung mit dem Reaktorinnenraum, bevorzugt Fermenterinnenraum bündig, bevorzugt flüssigkeitsdicht, bevorzugt fluiddicht, ab. Bevorzugt werden die Flüssigkeitsströmung und der Druck an der Membran in dem erfindungsgemäßen oder erfindungsgemäß bevorzugten Verfahren berücksichtigt. Alternativ kann der in situ Sensor auch als Durchflusssensor ausgebildet sein, welcher bevorzugt in einer Leitung, bevorzugt in ein Rohr, eingebaut ist.

Der in situ-Sensor kann auch in einem Rührer, einem Stromstörer oder in der Reaktorwand eines Reaktionsgefäßes integriert sein. Der in situ-Sensor wird bevorzugt als integraler Bestandteil eines Fermentationsprozess- oder Zellkulturprozess-Behälters, bevorzugt eines entsprechenden Einweg-Behälters verwendet. Der zur Aufnahme der Membran vorgesehene in situ-Sensor besteht vorzugsweise aus Kunststoff, bevorzugt PTFE, oder anderen Materialien. Die Membran kann aus demselben oder einem anderen Material aufgebaut sein.

Die vorliegende Erfindung betrifft bevorzugt eine Vorrichtung, wobei der mindestens eine Einlass für eine gasförmige Probe als Kapillareinlass ausgebildet ist.

Die vorliegende Erfindung betrifft bevorzugt eine Vorrichtung, wobei der Kapillareinlass eine mit Quarz beschichtete und beheizbare Kapillare aufweist.

Die vorliegende Erfindung betrifft bevorzugt eine Vorrichtung, wobei das Massenspektrometer eine Ionenquelle, einen Analysator und einen Detektor aufweist.

Die vorliegende Erfindung betrifft bevorzugt eine Vorrichtung, wobei das Massenspektrometer ein Quadrupol-Massenspektrometer ist.

Die vorliegende Erfindung betrifft bevorzugt eine Vorrichtung, wobei das Massenspektrometer ein Prozess-Massenspektrometer ist.

In einer bevorzugten Ausführungsform weist die Vorrichtung eine Membraneinrichtung zur Verflüchtigung von in einer flüssigen Probe vorhandenen Substanzen, eine Kapillare für den Einlass einer gasförmigen Probe und einen in situ-Sensor für den Einlass einer flüssigen Probe auf, bevorzugt sind diese Komponenten über ein Kreuzstück und schaltbaren Ventilen verbunden und automatisiert ansteuerbar. In einer bevorzugten Ausführungsform weist die Vorrichtung mindestens zwei Membraneinrichtungen zur Verflüchtigung von in einer flüssigen Probe vorhandenen Substanzen und eine Kapillare für den Einlass einer gasförmigen Probe, wobei mindestens eine Membraneinrichtung als in situ-Sensor für den Einlass einer flüssigen Probe ausgebildet ist.

Insbesondere ist die Vorrichtung als transportierbares und autarkes Modul konstruiert. Die vorliegende Vorrichtung liegt bevorzugt in einer transportierbaren Einhausung vor. Demgemäß kann die Vorrichtung in schon bestehende Prozessabläufe integriert werden.

Die vorliegende Vorrichtung ist insbesondere in der Lage, unterschiedliche Proben, sowohl gasförmige als auch flüssige Proben und Kalibriermedien, bevorzugt Kalibrierlösungen, einem massenspektrometrischen Analysieren oder Bestimmen von gasförmig vorliegenden Substanzen ohne Umbau der Vorrichtung zuzuführen.

Bevorzugt verfügt die Vorrichtung ebenfalls über einen Einlass für eine Säure oder eine Base. Durch die Zugabe einer Säure oder Base wird die Empfindlichkeit des Massenspektrometers gegenüber bestimmten Stoffen erhöht, indem je nach pKs-Wert der zu messenden Substanz ihre Flüchtigkeit durch Protonieren oder Deprotonieren verbessert wird. Insbesondere wird die Säure oder Base zunächst mittels einer Pumpe in die flüssige Probe eingebracht, insbesondere wenn die Membraneinrichtung in einem Bypass montiert ist. Insbesondere kann durch die Verwendung von zwei pulsationsarmen Pumpen das Einbringen einer Säure oder Base im Bypass ermöglicht werden.

Durch die vorliegende Vorrichtung kann ohne Umbaumaßnahmen abwechselnd sowohl aus einer flüssigen Probe als auch aus einer gasförmigen Probe eine massenspektrometrische Analyse oder Bestimmung von entsprechend flüchtigen Substanzen durchgeführt werden. Mittels mindestens einer eine Ventileinrichtung aufweisenden lösbaren Durchflusselementverbindung, auch als Flansch bezeichnet, können ein oder mehrere weitere Einlässe für gasförmige und/oder flüssige Proben bereitgestellt werden.

Die vorliegende Vorrichtung weist bevorzugt pro vorhandenes Durchflusselement mindestens ein Heizelement auf. Durch diese Heizelemente kann insbesondere für ein gleichmäßiges Temperaturprofil gesorgt werden, um so die Adsorption oder Kondensation der gasförmigen Substanzen an der Innenseite der Durchflusselemente, insbesondere der Leitung, verhindert werden.

Bevorzugt sind alle Durchflusselemente und Ventileinrichtungen, insbesondere alle Oberflächen, mit denen die gasförmig vorliegenden Substanzen in Kontakt kommen können, elektropoliert. Durch die Elektropolitur wird ebenfalls die Adsorption oder Kondensation der gasförmig vorliegenden Substanzen vermieden.

Die erfindungsgemäße oder erfindungsgemäß bevorzugte Vorrichtung weist insbesondere ein Steuerungssystem auf. Durch das Steuerungssystem ist eine Kommunikation mit dem Massenspektrometer, den Ventileinrichtungen, insbesondere den Ventilen, und den verbauten Pumpen möglich. Dadurch kann die Vorrichtung autark in einem breiten Anwendungsfeld eingesetzt werden.

Das Steuerungssystem der erfindungsgemäß bevorzugten Vorrichtung ermöglicht es insbesondere, das erfindungsgemäße Verfahren so zu steuern, dass zeitlich versetzt mindestens eine Substanz aus einer flüssigen Probe und mindestens eine Substanz aus einer gasförmigen Probe, insbesondere aus einem eine flüssige und gasförmige Phase enthaltenden Reaktionssystem, entnommen und nacheinander in gasförmigem Zustand einem massenspektrometrischen Analysieren der zu analysierenden Substanzen zugeführt werden.

Die in dem Massenspektrometer gemessenen Ionenströme, bevorzugt mit anderen Prozessparametern, werden bevorzugt auf das Steuerungssystem übertragen. Damit kann ein Druckabfall oder Druckanstieg detektiert und somit registriert werden und somit bei Verdacht auf eine Havarie, beispielsweise Flüssigkeitseinbruch, ein Teil oder alle Ventile in der Vorrichtung, insbesondere der Einlässe, geschlossen werden.

In dem Steuerungssystem sind bevorzugt mathematische Modelle hinterlegt, wodurch auf Basis einer automatischen Kalibrierung Ionenströme in Konzentrationen umgerechnet werden. Auf der Benutzeroberfläche des Steuerungssystems können die erforderlichen Parameter für das Durchführen des Verfahrens, wie die eingesetzten Edukte und die zu erwartenden Produkte, erfragt werden. Das Steuerungssystem verfügt bevorzugt über ein automatisiertes Kalibrierprogramm. Mit dem Steuerungssystem können bestimmte Verfahrensabläufe programmiert werden. Insbesondere können damit die Intervalle zwischen der Messung einer gasförmigen Probe, einer flüssigen Probe oder eines Kalibrierungsmediums festgelegt werden, insbesondere über die Steuerung der verschiedenen Ventile.

Insbesondere sind ebenfalls in der Vorrichtung mindestens eine Schnittstelle integriert, wodurch die vorliegend gemessenen massenspektrometrischen Werte an übergeordnete Steuersysteme, insbesondere Reaktorsteuersysteme übermittelt werden können. Ebenfalls ist es möglich, durch entsprechende Schnittstellen von anderen Messvorrichtungen gemessene Messwerte, insbesondere der pH-Wert, Sauerstoff- und Kohlenstoffdioxidpartialdrücke, Leitfähigkeit, Menge der zugegebenen Stoffe und optische Dichte in einem Reaktionsmedium übermittelt werden. Die erfindungsgemäße Vorrichtung umfasst daher insbesondere ein Steuerungssystem, welches Schnittstellen für weitere Geräte, zum Beispiel für die Aufnahme von Betriebsparametern, und für von weiteren Messvorrichtungen erhaltene Werte aufweist, beispielsweise für die, vorzugsweise qualitative und/oder quantitative, Bestimmung nicht-flüchtiger Substanzen in der flüssigen Probe oder für Detektoren zur Bestimmung des pH-Wertes, von Sauerstoff- und Kohlenstoffdioxidpartialdrücken, Leitfähigkeit, Menge der zugebenden Stoffe und/oder optischer Dichte.

Das erfindungsgemäß eingesetzte Steuerungssystem weist sich durch einen kompakten Aufbau aus. Das Steuerungssystem weist vorzugsweise Schnittstellen zur Ein- und Ausgabe von Messwerten sowie Prozessparametern, Schnittstellen zu Datenspeichern, Schnittstellen, die einen Fernzugriff inklusive eines Wartungsmodus und eines Wartungsservice ermöglichen, Schnittstellen zu Prozessleitsystemen, insbesondere zur Bestimmung von Leitwerten unter Einbinden weiterer Daten des Prozessleitsystems, Schnittstellen zu einem Kalibriermodul beziehungsweise weist ein integriertes Kalibriermodul auf, insbesondere zur automatischen Kalibrierung der zu messenden Komponenten unter Ansteuerung von Ventilen, Pumpen und Sensoren, insbesondere im Hinblick auf Ventilpositionen für den Stand der Kalibrierlösung und die Temperatur sowie mindestens eine Kommunikationseinrichtung zu dem Massenspektrometer selbst, den eingesetzten Pumpen insbesondere Hochvakuum- und Vorvakuumpumpen sowie, vorzugsweise über die Ventile, zu dem mindestens einen Einlass für eine flüssige Probe und dem mindestens einen Einlass für eine gasförmige Probe sowie gegebenenfalls zu weiteren Einlässen für weitere gas- oder flüssige Proben auf. In bevorzugter Ausführungsform weist das Steuerungssystem ein Gehäuse und ein Display auf, insbesondere einen Touchscreen.

Es werden bevorzugt weitere Messgrößen, bevorzugt die Lebendzellzahl von Mikroorganismen, bevorzugt über einen Softsensor geschätzt.

Unter dem Begriff "Softsensor" (aus den Worten "Software" und "Sensor" zusammengesetzt), auch als virtueller Sensor oder Sensorfusion bezeichnet, wird kein real existierender Sensor verstanden, sondern eine Abhängigkeitssimulation von stellvertretenden Messgrößen zu einer Zielgröße. Somit wird die Zielgröße nicht direkt gemessen, sondern anhand zu ihr korrelierender Messgrößen und eines Modells der Korrelation berechnet. Somit kann beispielsweise anhand von gasförmigen und/oder verflüchtigbaren Substanzen auf die Konzentration von nicht-flüchtigen Substanzen geschlossen werden, insbesondere wenn die Konzentration der nicht-flüchtigen Substanz aufgrund einer stattfindenden Reaktion mit der Konzentration der gasförmigen und/oder verflüchtigbaren Substanzen korreliert.

In einer besonders bevorzugten Ausführungsform weist die erfindungsgemäße Vorrichtung zum massenspektrometrischen Analysieren von in flüssigen und gasförmigen Proben vorhandenen Substanzen aa) ein Massenspektrometer, bb) mindestens einen Einlass für eine flüssige Probe, cc) mindestens einen Einlass für eine gasförmige Probe, wobei dem mindestens einen Einlass für eine flüssige Probe ein erstes Durchflusselement und dem mindestens einen Einlass für eine gasförmige Probe ein zweites Durchflusselement jeweils nachgeschaltet sind, wobei das erste Durchflusselement verschieden von dem zweiten Durchflusselement ist, und wobei im Einlass eine hydrophobe zumindest teilweise poröse Membran angeordnet ist, und zusätzlich einen Detektor, für die, insbesondere quantitative und/oder qualitative, Bestimmung von in einer flüssigen Probe vorhandenen nichtflüchtigen Substanzen auf.

Die erfindungsgemäße Vorrichtung ist so bevorzugt in der Lage, massenspektrometrisch einerseits die flüchtigen Substanzen in der flüssigen und/oder gasförmigen Phase eines Reaktionssystems zu erfassen und andererseits mittels des Detektors für die qualitative und/oder quantitative Erfassung in der flüssigen Phase des Systems vorhandener nichtflüchtiger Substanzen diese nicht-flüchtigen Substanzen zu ermitteln.

In einer bevorzugten Ausführungsform ist der Detektor ein optischer Detektor, ein Detektor für elektromagnetische Strahlung oder ein Ultraschalldetektor.

Vorteilhafterweise können mit der vorliegenden Vorrichtung in Echtzeit Reaktionen analysiert und aufgezeichnet werden.

Unter dem Begriff "in Echtzeit" wird verstanden, dass innerhalb von Sekunden, bevorzugt innerhalb einer Sekunde, die mindestens eine eingebrachte Substanz analysiert werden kann.

Darüber hinaus zeichnet sie sich durch eine hohe Robustheit, hohe Spezifizität und leichte Bedienbarkeit, einen geringen Wartungsaufwand, geringe Betriebskosten und die Möglichkeit, das Messsystem in bestehende Prozesse zu integrieren, aus.

Außerdem ist es mit der vorliegenden Vorrichtung möglich, Substanzen auch in sehr geringen Konzentrationen sowohl aus einer flüssigen als auch gasförmigen Probe mit vernachlässigbarem Zeitverzug zu messen.

Insbesondere im Hinblick auf die industrielle Biotechnologie zeichnen sich weitere Vorteile ab. Insbesondere kann die Produktkonzentration bei Fermentationen in Echtzeit durch Messung der entstehenden Produkte ohne größeren Aufwand und exakt bestimmt werden. Dementsprechend kann mit der Echtzeitmessung der Produkte und der Nebenprodukte die Fermentation hinsichtlich maximaler Produktbildungsrate optimiert und die Raum-Zeit-Ausbeute gesteigert werden. Zudem können mehrere Reaktoren an die erfindungsgemäße Vorrichtung angebunden werden.

Bei einer Abgasanalytik in Bioprozessen wird häufig die Messung der Partialdrücke von CO₂ und O₂ gemessen, um auf das Zellwachstum und Produktbildungsrate rückschließen zu können. Durch den erfindungsgemäßen Aufbau, insbesondere durch das Verfahren und die Vorrichtung, kann die gesamte Zusammensetzung des Abgases einschließlich der Produkte gemessen werden. Zudem kann durch den Einlass für die flüssige Probe die gasgelöste Konzentration von CO₂ und Sauerstoff und alle anderen flüchtigen Substanzen aus der Fermentationsbrühe gemessen werden.

Die Vorrichtung ist dazu geeignet, unterschiedlichste Produkte zu messen, und kann daher auch für unterschiedliche Produktionsprozesse verwendet werden, die entsprechend einer jahreszeitlich bedingten Rohstoffverfügbarkeit und/oder einer Kundennachfrage wechseln. Dementsprechend kann die vorliegende Vorrichtung als zugeschnittenes, in Produktionsanlagen integriertes Analysegerät implementiert werden.

Darüber hinaus ist neben der Prozessüberwachung auch die Kontrolle der Produktqualität wichtig. In der Biotechnologie kommt es durch unterschiedliche Stoffwechselwege zu Verunreinigungen im Produkt. Beispielsweise ist Ethanol durch Methanol, Acetaldehyd, Ethylacetat und Diacetyl verunreinigt. Mit der vorliegenden Vorrichtung können diese Verunreinigungen nachgewiesen werden. Sie kann daher als Analysegerät für den Nachweis von Verunreinigung durch flüchtige Stoffe, das heißt zur Qualitätskontrolle, verwendet werden. Die erfindungsgemäße Vorrichtung kann daher bevorzugt bei der Bierherstellung eingesetzt werden.

Die vorliegende Vorrichtung kann im Bereich der Forschung und Entwicklung, Prozessüberwachung aller Arten von Prozessmedien mit flüchtigen Komponenten bis zur Qualitätskontrolle, beispielsweise in den Branchen der Chemischen Industrie, Petrochemie, Biotechnologie, Pharmazie, Medizintechnik und Lebensmittelindustrie, eingesetzt werden. Sie kann in Labor- und/oder Pilotanlagen, Produktionsstätten eingesetzt werden, insbesondere zur Optimierung des dort ablaufenden Produktionsprozesses. Sie kann ebenfalls in Produktionen in modularen und flexiblen Anlagen eingesetzt werden

Durch die Möglichkeit mit der vorliegenden Vorrichtung auch Spurenstoffe nachzuweisen, ist diese Vorrichtung auch zur Qualitätssicherung in sensiblen Produktionsbereichen sowie zur Überwachung von Trinkwasser und/oder Abwasser geeignet. Insbesondere ist es mit der vorliegenden Vorrichtung möglich, alle flüchtigen Komponenten aus einer flüssigen, bevorzugt wässrigen als auch aus gasförmigen Proben zu messen und dabei Konzentrationsänderungen über acht Dekaden vom unteren ppb-Bereich bis in den hohen Potenzbereich aufzeichnen zu können. Des Weiteren weist die Vorrichtung geringe Ansprechzeiten und die Möglichkeit, bis zu 30, bevorzugt 20 Stoffe gleichzeitig zu messen, auf.

Insbesondere kann die vorliegende Vorrichtung in der industriellen Biotechnologie und in der biobasierten Produktion als Messgerät zur Prozessanalyse eingesetzt werden. Insbesondere kann die Vorrichtung bei enzymatischen Prozessen, beispielsweise bei der Herstellung von Butandiol, Propandiol, Bersteinsäure, Ethanol aus Lignocellulose, Butanol, Polyolen, Acrylsäurebutylester, Thiolen, Estern, Milchsäure eingesetzt werden.

Ebenfalls kann die Vorrichtung in der Medizintechnik eingesetzt werden, um beispielsweise die Gaszusammensetzung der Atemluft, die Gasemission der Haut und alle flüchtigen Bestandteile direkt aus dem Blut messen zu können.

Die Beschreibung des Verfahrens zum massenspektrometrischen Analysieren, kurz auch als Analyseverfahren bezeichnet, und die Beschreibung der Vorrichtung zum massenspektrometrischen Analysieren, kurz auch als Analysevorrichtung bezeichnet, sind komplementär zueinander zu verstehen. Verfahrensschritte des Analyseverfahrens, die explizit oder implizit in Zusammenhang mit der Analysevorrichtung beschrieben wurden, sind bevorzugt einzeln oder miteinander kombiniert Schritte einer bevorzugten Ausführungsform des Analyseverfahrens. Merkmale der Analysevorrichtung, die explizit oder implizit in Zusammenhang mit dem Analyseverfahren beschrieben wurden, sind bevorzugt einzeln oder miteinander kombiniert Merkmale eines bevorzugten Ausführungsbeispiels der Analysevorrichtung. Diese zeichnet sich vorzugsweise durch wenigstens ein Merkmal aus, welches durch wenigstens einen Schritt einer bevorzugten Ausführungsform des Analyseverfahrens bedingt ist. Das Analyseverfahren zeichnet sich bevorzugt durch wenigstens einen Verfahrensschritt aus, der durch wenigstens ein Merkmal der Analysevorrichtung bedingt ist.

Die Erfindung wird im Folgenden anhand der nachfolgenden fünf Zeichnungen näher erläutert. Dabei zeigen
- Figur 1: eine schematische Darstellung einer erfindungsgemäß bevorzugten Vorrichtung zur massenspektrometrischen Analyse von sowohl flüssigen als auch gasförmigen Proben,
- Figur 2: einen als Ingoldstutzen ausgebildeten in situ-Sensor gemäß der vorliegenden Erfindung,
- Figur 3: Ionenströme (IC) in [A] von verschiedenen Substanzen über die Zeit t in [min] bei der Veraschung einer Carbonfaser,
- Figur 4: Ionenströme (IC) in [A] von verschiedenen in einem sogenannten Reed-Schalter vorliegenden Gasen über die Zeit t in [s],
- Figur 5: eine gemessene Enzymkinetik und eine mathematische Modellierung anhand einer hyperbolischen Geschwindigkeitsgleichung, und
- Figur 6: eine schematische Darstellung einer erfindungsgemäß bevorzugten alternativen Vorrichtung zur massenspektrometrischen Analyse von sowohl flüssigen als auch gasförmigen Proben.

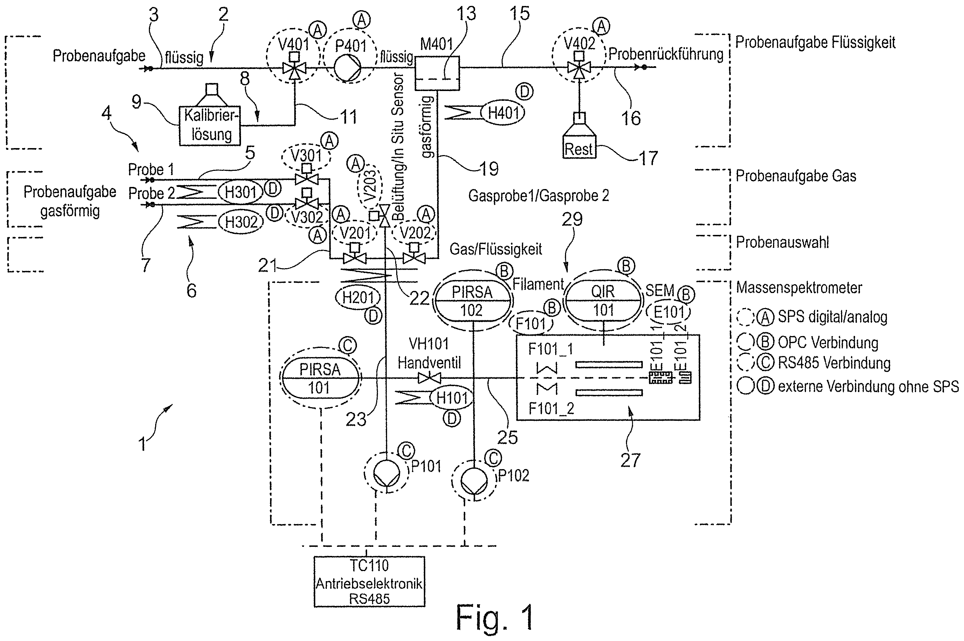

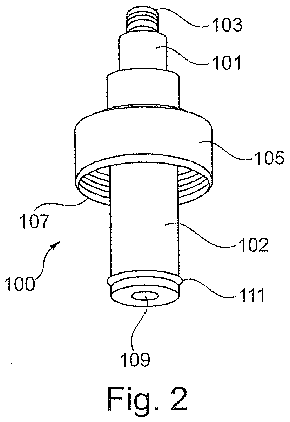

Insbesondere zeigt Figur 1 eine Vorrichtung 1, bei der über einen eine Leitung 3 aufweisenden Einlass 2 eine flüssige Probe aus einem Probeentnahmeraum und über Leitungen 5 und 7 aufweisende Einlässe 4, 6 jeweils eine gasförmige Probe eingebracht werden können. Alternativ oder zusätzlich kann eine in einem Behälter 9 bevorratete Kalibrierlösung über einen eine Leitung 11 aufweisenden Einlass 8 eingebracht werden. Die flüssige Probe oder die Kalibrierlösung werden mittels einer Pumpe P401 einer Membraneinrichtung M401 zugeführt. Die Pumpe P401 zeichnet sich durch eine Flussrate von 0 bis 50 Milliliter pro Minute, bevorzugt 10 Milliliter pro Minute, aus und kann damit einen Systemdruck von 0 bis 400 bar erzeugen. Über ein, bevorzugt pneumatisches, Ventil V401 kann wahlweise die flüssige Probe über die Leitung 3 oder die Kalibrierlösung über die Leitung 11 mittels der Pumpe P401 in flüssiger Form der Membraneinrichtung M401 zugeführt werden. Die flüssige Probe oder die Kalibrierlösung strömt parallel zu einer in der Membraneinrichtung M401 vorhandenen Membran 13 vorbei. Die Membran 13 ist erfindungsgemäßeine hydrophobe, zumindest teilweise poröse Membran, mithilfe derer flüchtige Substanzen, welche in der Kalibrierlösung oder der flüssigen Probe vorhanden sind, in einen gasförmigen Aggregatszustand überführt werden können. Der nicht verflüchtige oder zur Benetzung der Membran 13 dienende Flüssigkeitsanteil wird über eine Leitung 15 entweder in einen Behälter 17 abgeleitet oder über eine Leitung 16 in den Probenentnahmeraum zurückgeführt. Mit einem, bevorzugt pneumatischen, Ventil V402 kann dies gesteuert werden. Die verflüchtigten Substanzen der flüssigen Probe oder der Kalibrierlösung liegen gasförmig in einem ersten Durchflusselement 19, bevorzugt einer Leitung 19, vor. Das erste Durchflusselement 19 ist bevorzugt durch ein Heizelement H401 beheizbar. Die für die gasförmigen Proben vorgesehenen Leitungen 5 und 7 sind ebenfalls über Heizelemente H301 und H302 beheizbar. Die gasförmigen Proben können wahlweise über ein, bevorzugt pneumatisches, Ventil V301 oder ein, bevorzugt pneumatisches, Ventil V302 in ein zweites Durchflusselement 21, bevorzugt eine Leitung 21, eingebracht werden. Dem zweiten Durchflusselement 21 ist bevorzugt ebenfalls ein Heizelement zugeordnet. Durch ein, bevorzugt pneumatisches, Ventil V201 können über das Durchflusselement 21 oder durch ein, bevorzugt pneumatisches, Ventil V202 über das Durchflusselement 19 die gasförmig vorliegenden Substanzen in ein drittes Durchflusselement 23 eingebracht werden. Das Einbringen geschieht einerseits durch entsprechendes Schalten und Öffnen der Ventile V201 und V202 und andererseits zusätzlich durch die von einer Pumpe P101 erzeugte Sogwirkung. Durch die Pumpe P101 wird insbesondere ein Vorvakuum, das heißt ein Druck von 0,01 bis 0,5 mbar liegt vor, erzeugt. Ein weiteres, mit dem dritten Durchflusselement in Fluidkontakt stehendes Durchflusselement 22 kann einerseits als Belüftung oder zum Anschluss eines in Figur 2 gezeigten in situ-Sensor verwendet werden. Ein, bevorzugt pneumatisches, Ventil V203 verschließt in einer Schaltstellung das Durchflusselement 22 und stellt in einer anderen Schaltstellung eine Fluidverbindung nach außen oder zu dem in situ Sensor her. Dem dritten Durchflusselement 23 ist zudem ein PIRSA-Druckmesser PIRSA101 (P = Druck; I = Anzeige; R = Aufnahme; S = schaltbar; A = Alarm) zugeordnet. Über ein Handventil VH101 gelangen die gasförmigen Stoffe von dem dritten Durchflusselement 23 in ein viertes Durchflusselement 25. Das Handventil VH101 kann auch als über eine Steuerung bedienbares Ventil ausgebildet sein. Dem vierten Durchflusselement 25 ist eine Pumpe P102 zugeordnet, die insbesondere zur Erzeugung von Hochvakuum, das heißt zum Erzeugen eines Vakuums mit einem Druck von 10⁻⁵ mbar oder weniger geeignet ist. Ebenfalls ist diesem Durchflusselement 25 ein PIRSA-Druckmesser PIRSA102 zugeordnet. Die PIRSA-Druckmesser PIRSA101 und PIRSA102 dienen insbesondere dazu, um den in den dritten und vierten Durchflusselementen 23 und 25 vorliegenden Druck zu messen, aufzuzeichnen und gegebenenfalls Alarm auszulösen, falls sich der Ist-Druck von einem voreingestellten Solldruck unterscheidet. Dem Handventil VH101 ist ein Heizelement H101 zugeordnet. Alle Heizelemente H101, H201, H301, H302, H401 dienen insbesondere dazu, ein Beschlagen, das heißt Kondensieren oder Adsorbieren der gasförmig vorliegenden Substanzen an den entsprechenden Durchflusselementen zu verhindern. Die in dem vierten Durchflusselement 25 vorliegenden gasförmigen Substanzen gelangen anschließend zu einem in einem Massenspektrometer 29 vorliegenden Filament F101, wodurch die gasförmig vorliegenden Substanzen ionisiert werden. Die erzeugten Ionen werden durch ein statisches, elektrisches Feld beschleunigt und durchfliegen zentral vier parallel liegende Stabelektronen, deren Schnittpunkte mit einer Ebene senkrecht zur Zylinderachse ein Quadrat bilden, das sogenannte Quadrupol 27. Im Wechselfeld zwischen den Quadrupolstäben findet eine m/e-Selektierung statt, sodass jeweils nur Teilchen mit einer definierten Masse das Feld durchlaufen können. Anschließend treffen die Ionen in einem Detektor E101 mit Messverstärker auf, der den Ionenstrom misst und von der Software des angeschlossenen PCs zur Zählrate beziehungsweise zum Partialdruck umgerechnet wird. Der Detektor E101 ist ein Sekundärelektronenvervielfacher E101_1 (abgekürzt als SEM oder SEV). Darüber hinaus weist er einen Faraday-Auffänger E101_2 auf. Das Massenspektrometer 29 weist zudem einen QIR-Sensor QIR101 (Q = Menge, I = Anzeige, R = Aufzeichnung) auf. Außerdem sind der Druckmesser PIRSA101 und die Pumpen P101 und P102 jeweils über eine Schnittstelle RS485 mit einer Antriebselektronik verbunden.

Die mit dem Symbol A gekennzeichneten Elemente der Vorrichtung können mit einer digitalen oder analogen speicherprogrammierbaren Steuerung angesteuert werden. Die mit einem Symbol B gekennzeichneten Elemente weisen eine OPC-Verbindung auf. Die mit einem Symbol C gekennzeichneten Elemente haben eine RS485-Schnittstellenverbindung und die mit dem Symbol D gekennzeichneten Elemente haben eine externe Verbindung, aber keine Verbindung zur speicherprogrammierten Steuerung.

Figur 2 zeigt einen in situ-Sensor 100, der ein zylindrisches Innenteil 101 mit nicht sichtbaren Außengewinde und einer Hülse 102 mit nicht sichtbaren Innengewinde aufweist, wobei das zylindrische Innenteil 101 in die Hülse 102 eingeschraubt ist, wobei das Innengewinde und das Außengewinde zusammenwirken. Das Innenteil 101 weist zudem eine sich in Längsrichtung erstreckende nicht sichtbare Leitung, bevorzugt Bohrung, auf, wodurch eine Fluidverbindung zwischen dem flüssigen Medium oder Probe und dem Einlass der Vorrichtung für die flüssigen Proben vorliegt. Bevorzugt weist der in situ-Sensor 100 ein Heizelement auf, sodass der gesamte Sensor 100 beheizbar ist. Eine Membran 109 ist mithilfe einer passenden Dichtung, bevorzugt O-Ringdichtung, zwischen Innenteil 101 und der Hülse 102 eingespannt, bevorzugt durch Festschrauben der Hülse 102. Die Hülse 102 weist eine Öffnung, bevorzugt Bohrung, an diesem Membranende auf, sodass die flüssige Probe in ausreichender Menge bei bestimmungsgemäßer Verwendung die Membran benetzen kann. Diese Membran 109 ist bevorzugt so in dem in situ-Sensor 100 eingesetzt, dass sich eine, bevorzugt leichte, Wölbung nach außen, also in Richtung der Flüssigkeit oder Probe ergibt. Durch diese bevorzugte Wölbung verbessert sich die Verflüchtigung der in dem Medium vorliegenden flüchtigen Substanzen und/oder verringert sich die Biofilmbildung auf der Membran in einem Fermenter. Zur Verhinderung der Biofilmbildung ist bevorzugt alternativ oder zusätzlich ein Wischelement vorgesehen. Bevorzugt wird diese Membran 109 durch eine nicht sichtbare Scheibe, bevorzugt eine Sinterscheibe, gestützt, bevorzugt entgegen der Wölbung. Mittels einer ebenfalls vorhandenen, ein Gewinde 107 aufweisende Überwurfmutter 105 kann der in situ-Sensor bevorzugt an einem Reaktionsgefäß, bevorzugt an einem Fermenter befestigt werden und schließt bevorzugt über eine an der Hülse 102 außen anliegenden Ringdichtung 111 mit dem Reaktorinnenraum, bevorzugt Fermenterinnenraum bündig, bevorzugt flüssigkeitsdicht, bevorzugt fluiddicht, ab. An dem dem Membranende gegenüberliegenden Ende des Sensors 100 liegt ein Außengewinde 103 vor, mit dem der in situ-Sensor 100 mit der Vorrichtung 1, insbesondere mit dem Durchflusselement 19, bevorzugt unmittelbar, verbunden werden kann.

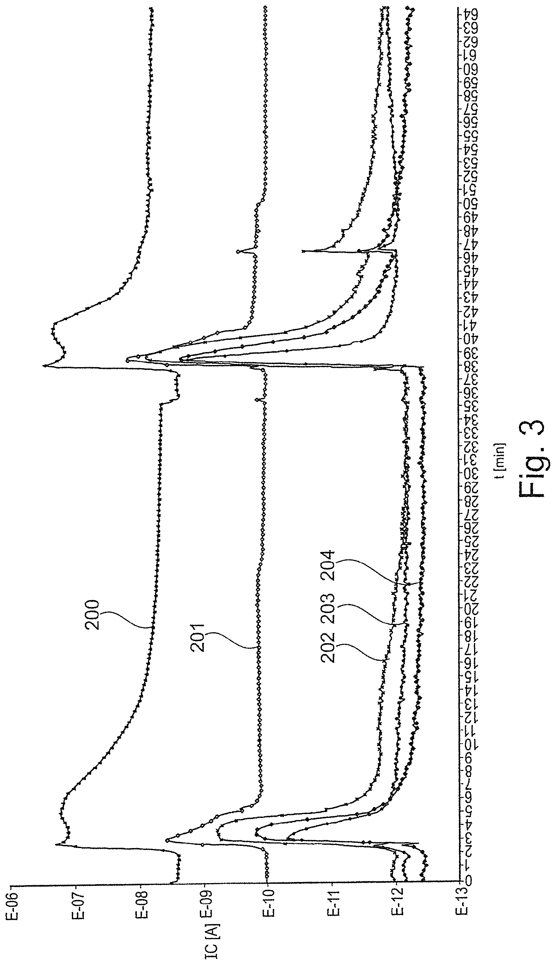

Figur 3 zeigt Ionenströme (IC) in [A] über die Zeit t in [min] bei der Veraschung einer Carbonfaser bei 700 °C. Dabei zeigt die Kurve 200 den Ionenstrom von CO₂, die Kurve 201 den Ionenstrom von Wasserstoff, die Kurve 202 von Benzol, die Kurve 203 von aliphatischen Kohlenwasserstoffen und die Kurve 204 von aromatischen Kohlenwasserstoffen.

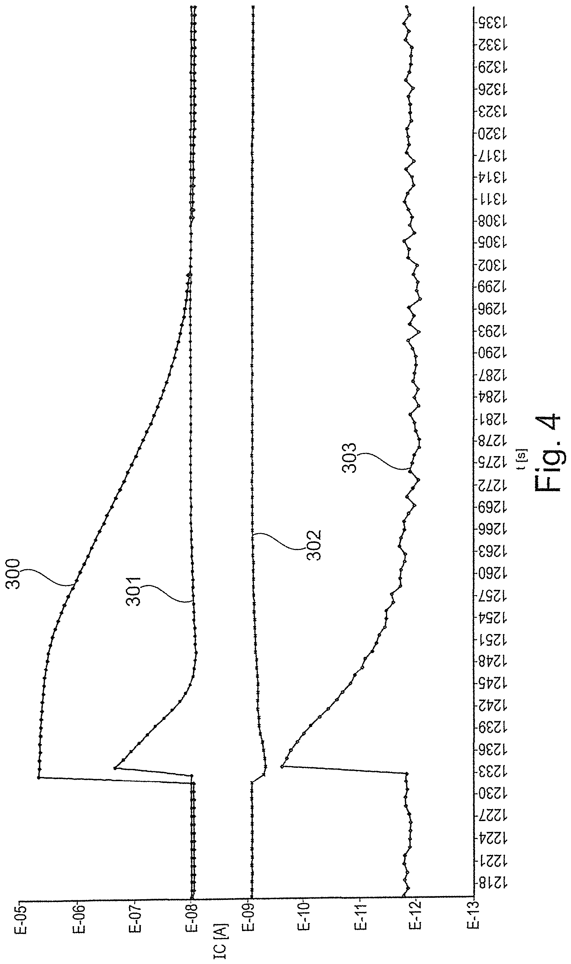

Figur 4 zeigt die Ionenströme (IC in [A]) über die Zeit t in [s] verschiedener in einem Reed-Schalter vorhandener Gase (Stickstoff (Kurve 300), Wasserstoff (Kurve 301), Sauerstoff (Kurve 302) und Helium (Kurve 303)). Zur Online-Messung der Gaszusammensetzung des Glasrohres des Reed-Schalters wurde dieses in situ direkt unter Vakuum mit einem Magneten gebrochen.

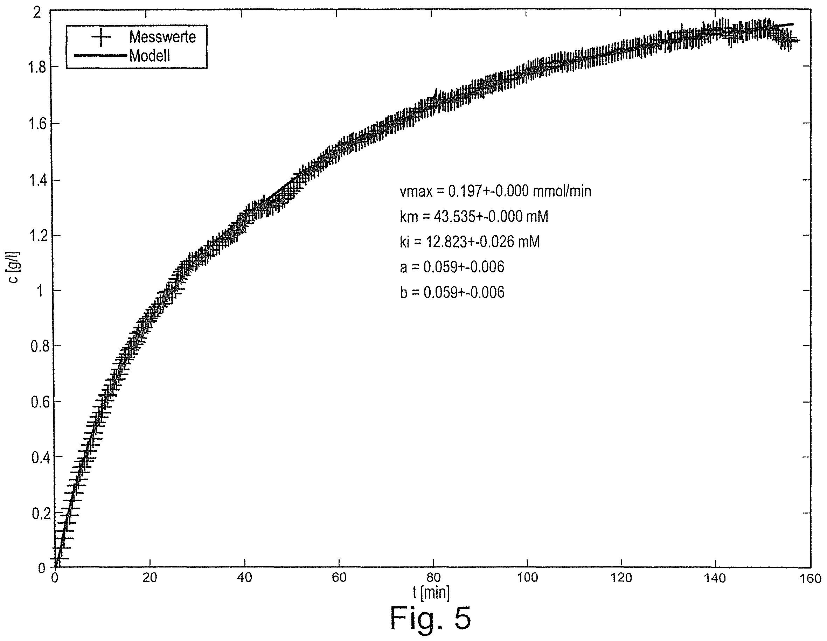

Figur 5 zeigt die Methanolkonzentration bei der enzymatischen Herstellung von Methanol und Ameisensäure aus Formaldehyd. Durch eine zuvor durchgeführte Kalibrierung des Massenspektrometers ist die Umrechnung der Ionenströme zu Konzentrationen möglich. Durch die hohe Datendichte der Kinetik ist eine mathematische Modellierung der Enzymreaktion möglich. Dementsprechend zeigt Figur 5 die Methanolkonzentration c in g/l über die fortschreitende Zeit t in Minuten.

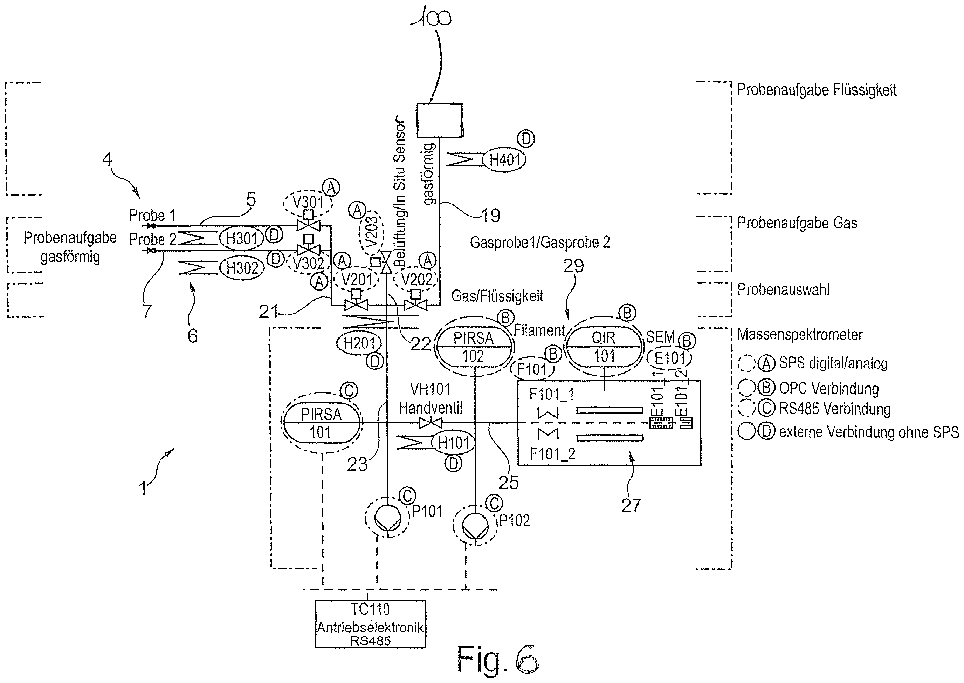

Figur 6 zeigt eine alternative Vorrichtung 1, die der in Figur 1 dargestellten Vorrichtung entspricht, wobei jedoch ein in situ-Sensor 100, eingebracht in einen nicht dargestellten Bioreaktor, den Einlass für eine flüssige Probe in das erste Durchflusselement 19 darstellt. Am in situ-Sensor 100 werden die flüchtigen, zu analysieren Substanzen aus der flüssigen Probe in den gasförmigen Zustand überführt und in das erste Durchflusselement 19 eingeleitet.

## Patentansprüche

1. Verfahren zum massenspektrometrischen Analysieren unter Nutzung einer Vorrichtung zum massenspektrometrischen Analysieren von in flüssigen und gasförmigen Proben vorhandenen Substanzen, umfassend die Schritte:
a) Einbringen mindestens einer Substanz aus einer flüssigen Probe in ein erstes Durchflusselement (19) einer Vorrichtung (1) durch Verflüchtigen der mindestens einen Substanz aus der flüssigen Probe an einer flüssigkeitsundurchlässigen und gasdurchlässigen Membran (13) und Einbringen mindestens einer Substanz einer gasförmigen Probe in ein zweites Durchflusselement (21) der Vorrichtung (1), wobei das erste Durchflusselement (19) von dem zweiten Durchflusselement (21) verschieden ist, sodass nach Einbringen der mindestens einen Substanz in das erste und zweite Durchflusselement (19, 21) die mindestens eine Substanz gasförmig in dem jeweiligen Durchflusselement vorliegt, und
b) massenspektrometrisches Analysieren der mindestens einen in Schritt a) gasförmig vorliegenden Substanz aus der flüssigen Probe und der mindestens einen in Schritt a) gasförmig vorliegenden Substanz aus der gasförmigen Probe.

2. Verfahren nach Anspruch 1, wobei in einem vor dem Schritt b) liegenden Schritt a1) die mindestens eine in Schritt a) gasförmig vorliegende Substanz aus der flüssigen Probe und die mindestens eine in Schritt a) gasförmig vorliegende aus der gasförmigen Probe in ein drittes Durchflusselement (23) der Vorrichtung (1) eingebracht werden, wobei in dem dritten Durchflusselement (23) ein Druck von 0,01 bis 0,5 mbar vorliegt.

3. Verfahren nach einem der vorhergehenden Ansprüche, wobei in einem vor dem Schritt b) liegenden Schritt a2) die mindestens eine in Schritt a) gasförmig vorliegende Substanz aus der flüssigen Probe und die mindestens eine in Schritt a) gasförmig vorliegende Substanz aus der gasförmigen Probe in ein viertes Durchflusselement (25) der Vorrichtung eingebracht werden, wobei in dem vierten Durchflusselement ein Druck von 10⁻⁵ mbar oder weniger vorliegt.

4. Verfahren nach einem der vorhergehenden Ansprüche, wobei das massenspektrometrische Analysieren gemäß Schritt b) die folgenden Schritte umfasst:
i) Ionisieren der mindestens einen gasförmig vorliegende Substanz aus der flüssigen Probe und der mindestens einen gasförmig vorliegende Substanz aus der gasförmigen Probe,
ii) Beschleunigen der in Schritt i) mindestens einen ionisierten Substanz,
iii) Selektieren der in Schritt ii) mindestens einen beschleunigten Substanz, und
iv) Detektieren der in Schritt iii) mindestens einen selektierten Substanz.

5. Verfahren nach einem der vorhergehenden Ansprüche, wobei das erste, zweite, dritte und/oder vierte Durchflusselement (19, 21, 23, 25) auf eine Temperatur von 60 bis 80 °C erhitzt wird.

6. Verfahren nach einem der vorhergehenden Ansprüche, wobei das Einbringen der mindestens einen Substanz aus der flüssigen Probe und der mindestens einen Substanz aus der gasförmigen Probe in das erste und zweite Durchflusselement (19, 21) derart gesteuert wird, dass in Schritt a1) und/oder a2) gleiche Mengen der mindestens einen gasförmigen Substanz in dem dritten Durchflusselement (23) vorliegen.

7. Verfahren nach einem der vorhergehenden Ansprüche, wobei der Druck in dem vierten Durchflusselement (25) detektiert wird.

8. Verfahren nach einem der vorhergehenden Ansprüche, wobei bei einem detektierten Druckanstieg in dem vierten Durchflusselement eine zwischen dem dritten und vierten Durchflusselement angeordnete ansteuerbare Ventileinrichtung geschlossen wird, sodass zwischen dem dritten und vierten Durchflusselement kein Fluidkontakt besteht.

9. Verfahren nach einem der vorhergehenden Ansprüche, wobei nicht-flüchtige Substanzen einer flüssigen Probe mittels eines Detektors zur Bestimmung von nicht-flüchtigen Substanzen in einer flüssigen Probe bestimmt werden.

10. Vorrichtung (1) zur Durchführung eines Verfahrens nach einem der Ansprüche 1 bis 9 zum massenspektrometrischen Analysieren von in flüssigen und gasförmigen Proben vorhandenen Substanzen, aufweisend
aa) ein Massenspektrometer (29),
bb) mindestens einen Einlass (2) für eine flüssige Probe und
cc) mindestens einen Einlass (4, 6) für eine gasförmige Probe,
wobei dem mindestens einen Einlass (2) für eine flüssige Probe ein erstes Durchflusselement (19) und dem mindestens einen Einlass (4, 6) für eine gasförmige Probe ein zweites Durchflusselement (21) jeweils nachgeschaltet sind und wobei das erste Durchflusselement (19) verschieden von dem zweiten Durchflusselement (21) ist, **dadurch gekennzeichnet, dass** im Einlass (2) eine hydrophobe zumindest teilweise poröse Membran (13) angeordnet ist.

11. Vorrichtung nach Anspruch 10, die zusätzlich mindestens einen Einlass (8) für ein Kalibriermedium aufweist.

12. Vorrichtung nach einem der Ansprüche 10 oder 11, die mindestens eine Ventileinrichtung (VH101, V201, V301, V401) aufweist, welche zwischen den Einlässen (2, 4, 6, 8) und dem Massenspektrometer (29) angeordnet ist.

13. Vorrichtung nach einem der Ansprüche 10 bis 12, wobei der mindestens eine Einlass (2) für eine flüssige Probe als in situ-Sensor ausgebildet ist.

14. Vorrichtung nach einem der Ansprüche 10 bis 13, wobei der mindestens eine Einlass (4, 6) für eine gasförmige Probe als Kapillareinlass ausgebildet ist.

15. Vorrichtung nach Anspruch 14, wobei der Kapillareinlass eine mit Quarz beschichtete und beheizbare Kapillare aufweist.

16. Vorrichtung nach einem der Ansprüche 10 bis 15, wobei das Massenspektrometer (29) eine Ionenquelle (F101), einen Analysator (27) und einen Detektor (E101) aufweist.

17. Vorrichtung nach einem der Ansprüche 10 bis 16, wobei das Massenspektrometer (29) ein Quadrupol-Massenspektrometer (29) ist.

18. Vorrichtung nach einem der Ansprüche 10 bis 17, wobei das Massenspektrometer (29) ein Prozess-Massenspektrometer (29) ist.

19. Vorrichtung nach einem der Ansprüche 10 bis 18, wobei die Vorrichtung zusätzlich einen Detektor für nicht-flüchtige Substanzen in einer flüssigen Probe aufweist.

## Claims

1. A method for mass spectrometric analysis using a device for mass spectrometric analysis of substances present in liquid and gaseous samples, comprising the steps of:
a) introducing at least one substance from a liquid sample into a first flow element (19) of a device (1) by volatilizing the at least one substance from the liquid sample at a liquid-impermeable and gas-permeable membrane (13) and introducing at least one substance of a gaseous sample into a second flow element (21) of the device (1), wherein the first flow element (19) is different from the second flow element (21), so that after introducing the at least one substance into the first and second flow elements (19, 21) the at least one substance is present gaseously in the respective flow element, and
b) mass spectrometric analysis of the at least one substance present gaseously in step a) from the liquid sample and of the at least one substance present gaseously in step a) from the gaseous sample.

2. The method according to claim 1, wherein in a step a1) prior to step b) the at least one substance present gaseously in step a) from the liquid sample and the at least one substance present gaseously in step a) from the gaseous sample are introduced into a third flow element (23) of the device (1), wherein a pressure of 0.01 to 0.5 mbar is present in the third flow element (23).

3. The method according to one of the preceding claims, wherein in a step a2) prior to step b) the at least one substance present gaseously in step a) from the liquid sample and the at least one substance present gaseously in step a) from the gaseous sample are introduced into a fourth flow element (25) of the device, wherein a pressure of 10⁻⁵ mbar or less is present in the fourth flow element.

4. The method according to one of the preceding claims, wherein the mass spectrometric analysis according to step b) comprises the following steps:
(i) ionising the at least one substance present gaseously from the liquid sample and the at least one substance present gaseously from the gaseous sample,
ii) accelerating the at least one substance ionised in step i),
iii) selecting the at least one substance accelerated in step ii), and
iv) detecting the at least one substance selected in step iii).

5. The method according to one of the preceding claims, wherein the first, second, third and/or fourth flow element (19, 21, 23, 25) is heated to a temperature of 60 to 80 °C.

6. The method according to one of the preceding claims, wherein the introducing of the at least one substance from the liquid sample and the at least one substance from the gaseous sample into the first and second flow element (19, 21) is controlled such that in step a1) and/or a2) equal amounts of the at least one gaseous substance are present in the third flow element (23).

7. The method according to one of the preceding claims, wherein the pressure in the fourth flow element (25) is detected.

8. The method according to one of the preceding claims, wherein upon a detected pressure increase in the fourth flow element, a controllable valve device arranged between the third and fourth flow elements is closed so that there is no fluid contact between the third and fourth flow elements.

9. The method according to one of the preceding claims, wherein non-volatile substances of a liquid sample are determined by means of a detector for determining non-volatile substances in a liquid sample.

10. A device (1) for carrying out a method according to one of claims 1 to 9 for mass spectrometric analysis of substances present in liquid and gaseous samples, having
aa) a mass spectrometer (29),
bb) at least one inlet (2) for a liquid sample and
cc) at least one inlet (4, 6) for a gaseous sample,
wherein a first flow element (19) is connected downstream of the at least one inlet (2) for a liquid sample and a second flow element (21) is connected downstream of the at least one inlet (4, 6) for a gaseous sample, and wherein the first flow element (19) is different from the second flow element (21), **characterised in that** a hydrophobic, at least partially porous membrane (13) is arranged in the inlet (2).

11. The device according to claim 10, which additionally has at least one inlet (8) for a calibration medium.

12. The device according to one of claims 10 or 11, which has at least one valve device (VH101, V201, V301, V401) arranged between the inlets (2, 4, 6, 8) and the mass spectrometer (29).

13. The device according to one of claims 10 to 12, wherein the at least one inlet (2) for a liquid sample is formed as an in-situ sensor.

14. The device according to one of claims 10 to 13, wherein the at least one inlet (4, 6) for a gaseous sample is formed as a capillary inlet.

15. The device according to claim 14, wherein the capillary inlet has a quartz-coated and heatable capillary.

16. The device according to one of claims 10 to 15, wherein the mass spectrometer (29) has an ion source (F101), an analyser (27) and a detector (E101).

17. The device according to one of claims 10 to 16, wherein the mass spectrometer (29) is a quadrupole mass spectrometer (29).

18. The device according to one of claims 10 to 17, wherein the mass spectrometer (29) is a process mass spectrometer (29).

19. The device according to one of claims 10 to 18, wherein the device additionally has a detector for non-volatile substances in a liquid sample.

## Revendications

1. Un procédé d'analyse par spectrométrie de masse utilisant un dispositif d'analyse par spectrométrie de masse de substances présentes dans des échantillons liquides et gazeux, comprenant les étapes suivantes :
a) introduire au moins une substance d'un échantillon liquide dans un premier élément d'écoulement (19) d'un dispositif (1) en volatilisant l'au moins une substance de l'échantillon liquide au niveau d'une membrane imperméable aux liquides et perméable aux gaz (13) et introduire l'au moins une substance d'un échantillon gazeux dans un second élément d'écoulement (21) du dispositif (1), dans lequel le premier élément d'écoulement (19) est différent du second élément d'écoulement (21), de manière à ce qu'après introduire l'au moins une substance dans le premier et le second éléments d'écoulement (19, 21), l'au moins une substance soit présente sous forme gazeuse dans l'élément d'écoulement respectif, et
b) analyse par spectrométrie de masse de l'au moins une substance présente sous forme gazeuse à l'étape a) de l'échantillon liquide et de l'au moins une substance présente sous forme gazeuse à l'étape a) de l'échantillon gazeux.

2. Le procédé selon la revendication 1, dans lequel à une étape a1) avant l'étape b), l'au moins une substance présente sous forme gazeuse à l'étape a) de l'échantillon liquide et l'au moins une substance présente sous forme gazeuse à l'étape a) de l'échantillon gazeux sont introduites dans un troisième élément d'écoulement (23) du dispositif (1), dans lequel une pression de 0,01 à 0,5 mbar est présente dans le troisième élément d'écoulement (23).

3. Le procédé selon l'une des revendications précédentes, dans lequel, à une étape a2) avant l'étape b), l'au moins une substance présente sous forme gazeuse à l'étape a) de l'échantillon liquide et l'au moins une substance présente sous forme gazeuse à l'étape a) de l'échantillon gazeux sont introduites dans un quatrième élément d'écoulement (25) du dispositif, dans lequel une pression inférieure ou égale à 10⁻⁵ mbar est présente dans le quatrième élément d'écoulement.

4. Le procédé selon l'une des revendications précédentes, dans lequel l'analyse par spectrométrie de masse selon l'étape b) comprend les étapes suivantes :
(i) ioniser l'au moins une substance présente sous forme gazeuse de l'échantillon liquide et l'au moins une substance présente sous forme gazeuse de l'échantillon gazeux,
ii) accélérer l'au moins une substance ionisée à l'étape i),
iii) choisir l'au moins une substance accélérée à l'étape ii), et
iv) détecter l'au moins une substance choisie à l'étape iii).

5. Le procédé selon l'une des revendications précédentes, dans lequel le premier, le deuxième, le troisième et/ou le quatrième élément d'écoulement (19, 21, 23, 25) est chauffé à une température de 60 à 80 °C.

6. Le procédé selon l'une des revendications précédentes, dans lequel l'introduction d'au moins une substance de l'échantillon liquide et d'au moins une substance de l'échantillon gazeux dans le premier et le deuxième élément d'écoulement (19, 21) est contrôlée de telle sorte qu'à l'étape a1) et/ou a2), des quantités égales d'au moins une substance gazeuse sont présentes dans le troisième élément d'écoulement (23).

7. Le procédé selon l'une des revendications précédentes, dans lequel on détecte la pression dans le quatrième élément d'écoulement (25).

8. Le procédé selon l'une des revendications précédentes, dans lequel lors d'une augmentation de pression détectée dans le quatrième élément d'écoulement, un dispositif de vanne contrôlable disposé entre les troisième et quatrième éléments d'écoulement est fermé de manière à ce qu'il n'y ait pas de contact de fluide entre les troisième et quatrième éléments d'écoulement.

9. Le procédé selon l'une des revendications précédentes, dans lequel les substances non volatiles d'un échantillon liquide sont déterminées au moyen d'un détecteur permettant de déterminer les substances non volatiles dans un échantillon liquide.

10. Un dispositif (1) pour la mise en oeuvre d'un procédé selon l'une des revendications 1 à 9 pour l'analyse par spectrométrie de masse de substances présentes dans des échantillons liquides et gazeux, ayant
aa) un spectromètre de masse (29),
bb) au moins une entrée (2) pour un échantillon liquide et
cc) au moins une entrée (4, 6) pour un échantillon gazeux,
dans lequel un premier élément d'écoulement (19) est connecté en aval de l'au moins une entrée (2) pour un échantillon liquide et un second élément d'écoulement (21) est connecté en aval de l'au moins une entrée (4, 6) pour un échantillon gazeux, et dans lequel le premier élément d'écoulement (19) est différent du second élément d'écoulement (21), **caractérisé en ce qu'**une membrane hydrophobe, au moins partiellement poreuse (13) est disposée dans l'entrée (2).

11. Le dispositif selon la revendication 10, qui a en outre au moins une entrée (8) pour un milieu de calibration.

12. Le dispositif selon l'une des revendications 10 ou 11, qui a au moins un dispositif de vanne (VH101, V201, V301, V401) disposé entre les entrées (2, 4, 6, 8) et le spectromètre de masse (29).

13. Le dispositif selon l'une des revendications 10 à 12, dans lequel l'au moins une entrée (2) pour un échantillon liquide est formée comme un capteur in-situ.

14. Le dispositif selon l'une des revendications 10 à 13, dans lequel l'au moins une entrée (4, 6) pour un échantillon gazeux est formée comme une entrée capillaire.

15. Le dispositif selon la revendication 14, dans lequel l'entrée capillaire a un capillaire enrobé de quartz et chauffable.

16. Le dispositif selon l'une des revendications 10 à 15, dans lequel le spectromètre de masse (29) a une source d'ions (F101), un analyseur (27) et un détecteur (E101).

17. Le dispositif selon l'une des revendications 10 à 16, dans lequel le spectromètre de masse (29) est un spectromètre de masse quadripolaire (29).

18. Le dispositif selon l'une des revendications 10 à 17, dans lequel le spectromètre de masse (29) est un spectromètre de masse à processus (29).

19. Le dispositif selon l'une des revendications 10 à 18, dans lequel le dispositif a en outre un détecteur de substances non volatiles dans un échantillon liquide.
